# EUROPEAN PATENT SPECIFICATION

(11) **EP 1 124 572 B1**
(45) Date of publication and mention of the grant of the patent: **10.09.2003**
(21) Application number: 99970605.4
(22) Date of filing: 22.10.1999
(51) Int. Cl.: A61K 38/43, A61K 38/21, A61K 38/17, A61K 48/00, A61K 39/395, G01N 33/50, C12Q 1/70, C12N 15/12, C12N 15/52, A61P 13/12, A01K 67/027, C07K 14/47, C12N 9/10, C07K 14/81

(54) **GENES AND PROTEINS PREDICTIVE AND THERAPEUTIC FOR RENAL DISEASE AND ASSOCIATED DISORDERS**
PRÄDIKTIVE UND THERAPEUTISCHE FÜR NIERENERKRANKUNGEN GENE UND PROTEINE
GENES ET PROTEINES DESTINES A LA PREVENTION ET THERAPIE DE MALADIES RENALES ET DE TROUBLES ASSOCIES

(30) Priority: 22.10.1998 US 177264
(43) Date of publication of application: 22.08.2001
(73) Proprietor: Curagen Corporation, New Haven, CT 06511 (US)
(72) Inventor: SHIMKETS, Richard, A., West Haven, CT 06516 (US)
(74) Representative: Schlich, George William
(86) International application number: US9924723
(87) International publication number: WO00023100

(56) References cited:
- WO-A-94/16724
- V.M. WATT ET AL.: "AMINO ACID SEQUENCE DEDUCED FROM A RAT KIDNEY cDNA SUGGESTS IT ENCODES THE Zn-PEPTIDASE AMINOPEPTIDASE N." JOURNAL OF BIOLOGICAL CHEMISTRY, vol. 264, no. 10, 5 April 1989 (1989-04-05), pages 5480-5487, XP002138184 BALTIMORE, MD, US cited in the application
- V. VLAHOVIC'ET AL.: "KIDNEY ECTOPEPTIDASES: STRUCTURE, FUNCTIONS AND CLINICAL SIGNIFICANCE." PATHOLOGIE BIOLOGIE, vol. 46, no. 10, December 1998 (1998-12), pages 779-786, XP000907210 PARIS, FR
- M.H.M. KUIJPERS ET AL.: "SPONTANEOUS HYPERTENSION AND HYPERTENSIVE RENAL DISEASE IN THE FAWN-HOODED RAT." THE BRITISH JOURNAL OF EXPERIMENTAL PATHOLOGY, vol. 65, no. 2, 1984, pages 181-190, XP000907274 LONDON, GB
- J. CHAO ET AL.: "Experimental Kallikrein Gene Therapy in Hypertension, Cardiovascular and Renal Diseases" PHARMACOLOGICAL RESEARCH,GB,ACADEMIC PRESS, LONDON, vol. 35, no. 6, 1997, pages 517-522-522, XP002099186 ISSN: 1043-6618
- J. CHAO ET AL.: "Human Kallikrein Gene Delivery Attenuates Hypertension, Cardiac Hypertrophy, and Renal Injury in Dahl Salt-Sensitive Rats" HUMAN GENE THERAPY, vol. 9, no. 9, 1 January 1998 (1998-01-01), pages 21-31-31, XP002099183 NEW YORK, US ISSN: 1043-0342
- D.M. BROWN ET AL.: "RENAL DISEASE SUSCEPTIBILITY AND HYPERTENSION ARE UNDER INDEPEDENT GENETIC CONTROL IN THE FAWN-HOODED RAT." NATURE GENETICS, vol. 12, January 1996 (1996-01), pages 44-51, XP000907186 NEW YORK, US

## Description

### RELATED APPLICATIONS

This application claims priority to USSN 09/177,264, filed October 22, 1998.

### FIELD OF THE INVENTION

The invention relates to nucleic acids and, more particularly, to genes expressed in renal disease.

### BACKGROUND OF THE INVENTION

Hypertension, hyperlipidemia and diabetes mellitus affect hundreds of millions of individuals world-wide and account for a significant fraction of morbidity and mortality, particularly among older individuals. These conditions can increase the risk for the development of conditions such as end-stage renal disease (ESRD), coronary heart disease (CHD) and stroke. For example, more than 70% of ESRD has been shown to be associated with hypertension.

Not all individuals suffering from hypertension, hyperlipidemia and diabetes mellitus develop ESRD, CHD or stroke. The underlying mechanism for this variability is currently unknown.

It has been suggested that the progression for ESRD, CHD or stroke is determined at least in part by one or more genetic factors.

### SUMMARY OF THE INVENTION

The present invention is based in part on the discovery of genes differentially expressed in an animal model of hypertension. Accordingly, disclosed herein is a set of 16 differentially-expressed genes [hereinafter "GENE SET"], as well as derivatives, fragments, analogs and homologs thereof, which were demonstrated to be differentially-expressed within awn-hooded rat (FHR) and IRL rodent models of renal disease, in comparison to control rat strain. These differentially-expressed genes are illustrated in Table 1 and include: Zn-peptidase (Aminopeptidase N); RT1.B-1(alpha) chain of the integral membrane protein; δ subunit of F1F0 ATPase; keratin 19; brain calbindin-d28k (CaBP28K); the inhibitor protein of metalloproteinase 3 (TIMP-3); integral membrane protein 1 (Itm1); isovaleryl-CoA dehydrogenase (IVD); *rab* GDI-β; IRPR (IFN-β); organic cation transporter (OCT2); bile mayaliculus domain-specific glycoprotein; L-arginine:glycine amidinotransferase; protein phosphatase 1-β (PP1-β); renal kallikrein and the p8 protein.

The present invention discloses therapeutic and/or prophylactic uses of Zn-peptidase (Aminopeptidase N) in the manufacture of medicaments for treating or preventing renal failure, platelet storage-pool disease, hypertension and/or other associated diseases or disorders.

Also disclosed by the present invention are methods useful for diagnosis, prognosis, and screening, by the detection of the differentially-expressed Zn-peptidase (Aminopeptidase N) protein and nucleic acid, as well as derivatives, fragments and analogs thereof. Diagnostic, prognostic and screening kits are also disclosed herein.

In an additional embodiment of the present invention, assays which screen for the therapeutic value of the Zn-peptidase (Aminopeptidase N) protein, nucleic acid and derivatives (and fragments and analogs thereof), as well as anti-Zn-peptidase (Aminopeptidase N) antibodies are also provided. Additionally, the present invention also discloses methods for the screening of modulators (*i.e*., activators or inhibitors) of the Zn-peptidase (Aminopeptidase N) protein or nucleic acid activity which affect renal disease, platelet storage-pool disease, hypertension and/or associated disease or disorders.

### DESCRIPTION OF THE FIGURES

In order that the present invention disclosed herein is better understood and appreciated, the following detailed description is set forth.
Figure 1: illustrates the differential-expression of IRPR (IFN-β) mRNA in the FHR, IRL and ACI (control) rodent strains at 8 and 32 weeks-of-age.
Figure 2: illustrates the confirmation of differential-expression of IRPR (IFN-β) mRNA in the FHR and ACI (control) rodent strains at 32 weeks-of-age by the Oligo Poisoning™ methodology.
Table 1: A list of genes which were demonstrated differentially-expressed with the FHR and IRL strains of rat, in comparison to the control ACT strain. GenBank Accession Number, common name, and fold modulation relative to the controls are provided.

### DETAILED DESCRIPTION OF THE INVENTION

There is disclosed herein a group of genes (hereinafter designated "GENE SET" genes) which are demonstrated to be differentially-expressed within experimental animal models (*i.e*., the FHR and IRL strains of rat) for renal failure. A total of 16 differentially-expressed GENE SET genes are characterized. In specific embodiments of the present invention, the Zn-peptidase (Aminopeptidase N) GENE SET protein or nucleic acid (and derivatives, fragments or analogs thereof), as well as anti-Zn-peptidase (Aminopeptidase N) antibodies are utilized for the manufacture of a medicament for the treatment or prevention of human platelet storage-pool disease, hypertension and, preferably, renal disease. Additionally, pharmaceutical compositions are also disclosed herein.

Other embodiments of the present invention relate to methods useful for diagnosis, prognosis and screening for existing, or future impairment of renal function by the detection of differential expression of human nucleic acid or amino acid sequences which are homologous to Zn-peptidase (Aminopeptidase N) for diagnostic purposes. In one specific embodiment, subjects are screened for dysregulation of Zn-peptidase (Aminopeptidase N)) genes.

Accordingly, in a first aspect, the invention provides use of Zn-peptidase (Aminopeptidase N) in the manufacture of a medicament for treating or preventing renal disease.

In second, third and fourth aspects of the invention there is provided the use of one or more of the following:
(a) antibodies specific for the Zn-peptidase (Aminopeptidase N) of claim 1;
(b) nucleic acids which encode the Zn-peptidase (Aminopeptidase N) of claim 1;
(c) anti-sense nucleic acid derivatives of the nucleic acids encoding the Zn-peptidase (Aminopeptidase N) of claim 1;
(d) antibodies specific for one or more of the nucleic acids which encode the Zn-peptidase (Aminopeptidase N) of claim 1; and
(e) antibodies specific for the anti-sense nucleic acid derivatives of the nucleic acids encoding the Zn-peptidase (Aminopeptidase N) of claim 1,
in the manufacture of a medicament for treating or preventing renal disease or platelet storage-pool disease or hypertension.

A further aspect of the invention provides a pharmaceutical composition comprising a therapeutically or prophylactically effective amount of the following:
(I)
   (a) an isolated, Zn-peptidase (Aminopeptidase N) protein as recited in claim 1;
   (b) antibodies specific for the Zn-peptidase (Aminopeptidase N) of claim 1;
   (c) nucleic acids encoding the Zn-peptidase (Aminopeptidase N) of claim 1;
   (d) anti-sense nucleic acid derivatives of the nucleic acids encoding the Zn-peptidase (Aminopeptidase N) of claim 1;
   (e) antibodies specific for one or more of said nucleic acids which encode the Zn-peptidase (Aminopeptidase N) of claim 1; and/or
   (f) antibodies specific for one or more of said anti-sense nucleic acid derivatives of the Zn-peptidase (Aminopeptidase N) of claim 1; and
(II) one or more antihypertensive drugs; and
(III) a pharmaceutically acceptable carrier.

In still further aspects, the invention provides methods of screening for a modulator of renal disease, platelet storage-pool disease, hypertension or related diseases or disorders of Zn-peptidase (Aminopeptidase N).

In yet a further aspect, the invention provides a recombinant, non-human animal comprising a non-native Zn-peptidase (Aminopeptidase N) gene sequence.

Also provided, in a further aspect of the invention, is a method useful for diagnosing the susceptibility or presence of renal disease, platelet storage-pool disease, hypertension or related diseases or disorders comprising:
(a) having provided a tissue sample from a subject;
(b) measuring the level of Zn-peptidase (Aminopeptidase N) in said subject or measuring the level of a nucleic acid encoding Zn-peptidase (Aminopeptidase N) in said subject; and
(c) comparing the amount of protein or nucleic acid in said test sample with the level of protein or nucleic acid in a control sample, wherein the control sample is taken from an individual not suffering or susceptible to platelet storage-pool disease, hypertension or related diseases or disorders,
wherein an alteration in the level of the protein or nucleic acid in the subject relative to the control indicates the subject is susceptible to or suffers from renal disease, platelet storage-pool disease, hypertension or related diseases or disorders.

### (1) Nucleic Acids and Encoded Proteins of the GENE SET

The GENE SET proteins (and derivative, fragments, analogs and homologs thereof) and the nucleic acids encoding the GENE SET proteins (and derivatives, fragments and analogs thereof) are provided herein. GENE SET proteins and nucleic acids may be obtained by any methodology known within the art. The GENE SET amino acid and nucleotide sequences for, *inter alia*, human, rat, hamster, dog, mouse, bovine, porcine, *Drosophila melanogaster*, Xenopus, horse, and dogfish are available in various public-access databases (*e.g*., GenBank, EMBL, and the like).

In the practice of the present invention, any eukaryotic cell may potentially serve as the nucleic acid source for the isolation of GENE SET nucleic acids. These GENE SET nucleic acids may be obtained by standard procedures known within the art including, but not limited to: (*i*) chemical synthesis; (*ii*) by cDNA cloning or (*iii*) by the cloning of genomic DNA, or fragments thereof, purified from the desired cell. See *e.g*., Sambrook, *et al*., 1989. *Molecular Cloning, A Laboratory Manual, 2d Ed*. (Cold Spring Harbor Laboratory Press, Cold Spring Harbor, New York); Glover, 1985. *DNA Cloning: A Practical Approach* (MRL Press, Ltd., Oxford, U.K.). It should be noted however, that clones which are derived from genomic DNA may contain regulatory and non-coding (intronic) DNA regions in addition to coding (exonic) regions; whereas clones which are derived from complementary DNA (cDNA) will contain only coding, exonic sequences.

In the molecular cloning of a gene from cDNA, cDNA is generated from total cellular RNA, or mRNA, by methods that are well-known within the art. The gene may also be obtained from genomic DNA, where DNA fragments are generated (*e.g*., using restriction enzymes or by mechanical shearing), some of which will possess the desired genomic sequence. The linear DNA fragments may then be separated according to size by standard techniques including, but not limited to, agarose and polyacrylamide gel electrophoresis and size-exclusion chromatography.

Once the DNA fragments are generated, identification of the specific DNA fragment containing all or a portion of the GENE SET gene may be accomplished in a number of ways. A preferred methodology for isolating a GENE SET gene is by amplification by the polymerase chain reaction (PCR), which may be used to amplify the desired GENE SET sequence in a genomic or cDNA library or, alternately, from genomic DNA or cDNA which has not been incorporated into a library. Oligonucleotide primers that hybridize to GENE SET sequences may be utilized as primers in PCR-mediated amplification reactions. The nucleic acid sequences of the oligonucleotide primers that are utilized are dependent upon the sequence of the specific fragment to be amplified, and may be readily ascertained by one who is skilled within the art.

Such synthetic oligonucleotides may be utilized as primers in PCR-mediated amplification of sequences of interest (RNA or DNA) which are derived, preferably, from a cDNA library. PCR may be preformed, for example, by use of a Perkin-Elmer Cetus thermal cycler and *Taq* polymerase (Gene Amp®). In the practice of the present invention, one may elect to synthesize several different degenerate primers for use in the PCR reactions. It is also possible to vary the stringency of hybridization conditions utilized in priming the PCR reactions, thus allowing for greater or lesser degrees of nucleotide sequence homology between the known GENE SET nucleotide sequence, and the nucleic acid sequence of a GENE SET homolog being isolated. In specific embodiments, low stringency conditions are preferred for cross species hybridization; whereas for same species hybridization, moderately stringent conditions are preferable.

Following the successful amplification of a segment of a GENE SET homolog, that segment of interest may be molecularly-cloned and sequenced, and subsequently utilized as a probe in the isolation of a complete cDNA or genomic clone. This, in turn, will permit the determination and isolation of the gene's complete nucleotide sequence. Alternately, PCR amplification may also be utilized to detect and quantitate GENE SET mRNA levels (*e.g*., for use in the diagnostic, prognostic and screening methods described Section 4, *infra*).

In one embodiment of the present invention, a portion of the GENE SET gene (derived from any species), or its specific mRNA, or a fragment thereof, may be isolated and labeled. The generated DNA fragments may then be screened by nucleic acid hybridization to a labeled probe (see *e.g.,* Benton & Davis, 1977. *Science* 196:180; Grunstein & Hogness, 1975. *Proc. Natl. Acad. Sci. U.S.A*. 72:3961) and those DNA fragments possessing substantial homology to the probe will hybridize. Alternately, in another embodiment, an oligonucleotide probe may be synthesized and labeled, and the generated DNA fragments may be screened by nucleic acid hybridization to the labeled oligonucleotide probe. In yet another embodiment, GENE SET nucleic acids may be also identified and isolated by expression-cloning using, for example, anti-GENE SET antibodies for the initial selection.

In alternative embodiments of the present invention, methods known within the art may be utilized to obtain GENE SET DNA by cloning or amplification. These methods include, but are not limited to: (*i*) chemically synthesizing the gene sequence itself from the known GENE SET sequence; (*ii*) generating a cDNA to the mRNA species which encodes the GENE SET protein and other methods within the scope of the invention. Once a clone has been obtained, its identity may be confirmed by nucleic acid sequencing, by any method well-known in the art, and compared to known GENE SET sequences. DNA sequence analysis methods include, but are not limited to: (*i*) the chemical method (see *e.g*., Maxam & Gilbert, 1980. *Meth. Enzymol.* 65:499-560); (*ii*) the dideoxynucleotide chain-termination method (see *e.g.,* Sanger, *et al*., 1977. *Proc. Natl. Acad. Sci. U.S.A*. 74:5463); (*iii*) the use of T₇ DNA polymerase (see *e.g.,* Tabor & Richardson, U.S. Patent No. 4,795,699); (*iv*) use of an automated DNA sequenator (*e.g*., Applied Biosystems; Foster City, CA) or (*v*) the method described in PCT Publication WO 97/15690, dated May 1, 1997 to Nandabalan, *et al*.

Nucleic acids, which are hybridizable to a GENE SET nucleic acid or to a nucleic acid encoding a GENE SET derivative, may be isolated by nucleic acid hybridization under conditions of low, high, or moderate stringency. By way of example, and not of limitation, hybridization procedures using such conditions of low stringency are as follows (see also e.g., Shilo & Weinberg, 1981. *Proc. Natl. Acad. Sci. USA* 78:6789-6792): filters containing immobilized DNA are pre-hybridized for 6 hours at 40°C in a solution containing: 35% formamide, 5X SSC, 50 mM Tris-HCl (pH 7.5), 5 mM EDTA, 0.1% PVP, 0.1% Ficoll, 1% BSA and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate and 5-20 x 10⁶ cpm ³²P-labeled probe. The filters are then incubated in hybridization mixture for 18-20 hours at 40°C, and washed for 1.5 hours at 55°C in a solution containing 2X SSC, 25 mM Tris-HCI (pH 7.4), 5 mM EDTA and 0.1% SDS. The wash solution is then replaced with fresh solution and the filters are incubated an additional 1.5 hours at 60°C. The filters are blotted dry and autoradiographed. If necessary, the filters are washed for a third time at 65-68°C and re-exposed to X-ray film. Other conditions of low stringency hybridization, which are well-known in the art (*e.g*., as employed for cross-species hybridizations), may also be employed in the practice of the present invention.

By way of example, and not of limitation, procedures using such conditions of moderate stringency hybridization are as follows: filters containing immobilized DNA are pre-hybridized for 6 hours at 55°C in a solution containing: 6X SSC, 5X Denhardt's solution, 0.5% SDS and 100 µg/ml denatured salmon sperm DNA. Hybridizations are performed in the same solution and 5-20 x 10⁶ cpm ³²P-labeled probe is used. The filters are incubated in hybridization mixture for 18-20 hours at 55°C, and then washed twice for at 37°C for 1 hour in a solution containing 2X SSC, 0.1% SDS. The filters are then blotted dry and autoradiographed. Other conditions of moderate stringency, which are well-known within the art, may be employed in the practice of the present invention.

Again, by way of example, and not of limitation, procedures using such conditions of high stringency hybridization are as follows: pre-hybridization of filters containing immobilized DNA is performed for 8 hours to overnight at 65°C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65°C in pre-hybridization mixture containing: 100 µg/ml denatured salmon sperm DNA and 5-20 x 10⁶ cpm of ³²P-labeled probe. Washing of filters is done at 37°C for 1 hour in a solution containing 2X SSC, 0.01% PVP, 0.01% Ficoll, and 0.01% BSA. This is then followed by a third wash in 0.1X SSC at 50°C for 45 minuets prior to autoradiography. Other conditions of high stringency hybridization, which are well-known within the art, may also be utilized in the practice of the present invention.

Nucleic acids which encode derivatives and analogs of GENE SET proteins, GENE SET anti-sense nucleic acids and primers that may be utilized to detect GENE SET alleles and GENE SET gene expression are additionally disclosed.

GENE SET proteins (and derivatives, analogs and fragments of GENE SET proteins) may be obtained by any method known within the art including, but not limited to, recombinant expression methods, purification from natural sources and chemical synthesis. In one embodiment of the present invention, GENE SET proteins may be obtained by recombinant protein expression techniques; wherein the GENE SET gene of interest (or portion thereof) is ligated into an appropriate cloning vector for subsequent expression within a particular host cell. A large number of vector-host systems are known with in the art, and include, but are not limited to: bacteriophages (*e.g*., λ bacteriophage and derivatives) or bacterial plasmids (*e.g*., pBR322 or pUC plasmid derivatives or the Bluescript® vector (Stratagene; La Jolla, CA)). The insertion into a cloning vector may, for example, be accomplished by ligating the DNA fragment into a cloning vector that possesses complementary, cohesive termini. However, if the complementary restriction site for the restriction endonuclease (RE) utilized to digest the insert DNA are not present within the cloning vector, the ends of the DNA molecules may be enzymatically modified (*e.g*., Klenow fragment of DNA polymerase I). Alternatively, any site desired may be produced by ligating oligonucleotide sequences (*i.e*., linkers) onto the DNA termini. Moreover, these ligated linkers may also comprise specific, chemically-synthesized oligonucleotides encoding RE recognition sequences, DNA sequence-specific DNA-binding protein binding sites, and the like. In an alternative embodiment of the present invention, the RE-digested vector and GENE SET gene of interest may be modified by homopolymeric tailing with terminal deoxynucleotidyl transferase (TdT). The recombinant molecules may then be introduced into host cells via transformation, transfection, infection, electroporation, etc., so that a plurality of copies of the gene sequence are generated.

In an alternative embodiment, the desired gene may be identified and isolated after insertion into a suitable cloning vector in a "shot-gun" approach. Enrichment for the desired gene (*e.g.*, by size fractionation) may be performed prior to the insertion of the sequence of interest into the cloning vector.

It should be noted that the molecular-cloning and expression of both cDNA and genomic sequences are within the scope of the present invention. In specific embodiments thereof, transformation of host cells with recombinant DNA molecules which incorporate the isolated GENE SET gene, cDNA or synthesized DNA sequence enables generation of multiple copies of the gene. Accordingly, the GENE SET gene sequence may be obtained in large quantities by *in vitro* culture of the transformants, isolating the recombinant DNA molecules from the transformants and, when necessary, retrieving the inserted gene from the isolated recombinant DNA.

The nucleotide sequence encoding a GENE SET protein (or a functionally-active analog, fragment or derivative thereof) may then be inserted into an appropriate expression vector (*i.e.*, a vector which contains the necessary (exogenous) regulatory elements for the transcription and translation of the inserted protein-coding sequence). In an alternate specific embodiment, the required transcriptional and translational regulatory signals may be supplied by the native (endogenous) GENE SET gene and/or its flanking regions . A variety of host-vector systems may be utilized to express the protein-coding sequence including, but are not limited to: (*i*) mammalian cell systems infected with virus (*e.g.,* vaccinia virus, adenovirus, etc.); (*ii*) insect cell systems infected with virus (*e*.*g*., baculovirus); (*iii*) microorganisms such as yeast containing yeast vectors or (*iv*) bacteria transformed with bacteriophage, DNA, plasmid DNA, or cosmid DNA. The expression elements of vectors vary in their strengths and specificities, and depending upon the host-vector system utilized, any one of a number of suitable transcription and translation elements may be utilized.

Similarly, any of the methods utilized for the insertion of DNA fragments into a vector may be used to construct expression vectors containing a chimeric gene consisting of appropriate transcriptional/translational control signals and the protein coding sequences. These methods include, but are not limited to, *in vitro* recombinant DNA and synthetic techniques and *in vivo* recombinants (genetic recombination). Expression of nucleic acid sequence encoding a GENE SET protein or peptide fragment may be regulated by a second nucleic acid sequence such that the GENE SET protein (or derivative, fragment or analog) is expressed in a host transformed with the recombinant DNA molecule. For example, expression of a GENE SET protein may be controlled by any promoter/enhancer element known within the art. In a specific embodiment of the present invention, the promoter is not native to the genes for the Zn-peptidase (Aminopeptidase N) protein. Promoters which may be utilized include, but are not limited to: (*i*) the SV40 early promoter (see *e.g.,* Bernoist & Chambon, 1981. *Nature* 290:304-310); (*ii*) the promoter contained in the 3'-terminus long terminal repeat of Rous sarcoma virus (see *e.g*., Yamamoto, *et al*., 1980. *Cell* 22:787-797); (*iii*) the Herpesvirus thymidine kinase promoter (see *e.g*., Wagner, *et al*., 1981. *Proc. Natl. Acad. Sci*. *USA* 78:1441-1445); (*iv*) the regulatory sequences of the metallothionein gene (see *e*.*g*., Brinster, *et al*., 1982. *Nature* 296:39-42); (*v*) prokaryotic expression vectors such as the β-lactamase promoter (see *e.g.,* Villa-Kamaroff, *et al*., 1978. *Proc. Natl*. *Acad*. *Sci*. *USA* 75:3727-3731) or (*vi*) the *tac* promoter (see *e.g.,* DeBoer, *et al*., 1983. *Proc. Natl. Acad. Sci*. *USA* 80:21-25. In addition, animal transcriptional control regions which exhibit tissue specificity and have been utilized in transgenic animals may also be utilized. These transcriptional control regions include, but are not limited to: (i) the elastase I gene control region which is active in pancreatic acinar cells (see *e.g*., Swift, *et al*., 1984. *Cell* 38:639-646; (*ii*) the insulin gene control region which is active in pancreatic β-cells (see *e.g.,* Hanahan, *et al*., 1985. *Nature* 315:115-122); (*iii*) the immunoglobulin gene control region which is active in lymphoid cells (see *e.g.,* Alexander, *et al*., 1987. *Mol. Cell Biol.* 7:1436-1444); (*iv*) the mouse mammary tumor virus control region which is active in testicular, breast, lymphoid and mast cells (see *e.g*., Leder, *et al*., 1986. *Cell* 45:485-495); (v) the α-fetoprotein gene control region which is active in liver (see *e.g.,* Krumlauf, *et al*., 1985. *Mol. Cell. Biol.* 5:1639-1648); (*vi*) the β-globin gene control region which is active in myeloid cells (see *e.g*., Kollias, *et al*., 1986. *Cell* 46:89-94) and *(vii)* the myosin light chain-2 gene control region which is active in skeletal muscle (see *e.g*., Sani, 1985. *Nature* 314:283-286).

In a specific embodiment of the present invention, a vector is utilized which comprises: (*i*) a promoter operably-linked to nucleic acid sequences encoding the Zn-peptidase (Aminopeptidase N) protein, or a fragment, derivative or homolog thereof; (*ii*) one or more origins of replication and optionally, (*iii*) one or more selectable markers *(e.g.,* an antibiotic resistance gene).

In a preferred embodiment of the present invention, a vector is utilized which comprises a promoter operably-linked to nucleic acid sequences encoding a Zn-peptidase (Aminopeptidase N) protein, one or more origins of replication, and one or more selectable markers. For example, in a specific embodiment, an expression construct is made by subcloning a Zn-peptidase (Aminopeptidase N) coding sequence into the *Eco*RI restriction site of each of the three pGEX vectors (Glutathione S-Transferase expression vectors; Smith & Johnson, 1988. *Gene* 7:31-40), thus allowing the expression of the Zn-peptidase (Aminopeptidase N) protein product from the subclone in the correct reading frame.

In specific embodiments of the present invention, expression vectors containing Zn-peptidase (Aminopeptidase N) gene inserts may be identified by three general approaches: (*i*) nucleic acid hybridization, (*ii*) presence or absence of "marker" gene functions, and (*iii*) expression of inserted sequences. In the first approach, the presence of a Zn-peptidase (Aminopeptidase N) gene inserted in an expression vector may be detected by nucleic acid hybridization using probes comprising sequences that are homologous to an inserted Zn-peptidase (Aminopeptidase N) gene. In the second approach, the recombinant vector/host system may be identified and selected based upon the presence or absence of certain "marker" gene functions (*e*.*g*., thymidine kinase activity, resistance to antibiotics, transformation phenotype, occlusion body formation in baculovirus, and the like) caused by the insertion of a Zn-peptidase (Aminopeptidase N) gene in the vector. For example, if the Zn-peptidase (Aminopeptidase N) gene is inserted within the marker gene sequence of the vector, recombinants containing the Zn-peptidase (Amiopeptidase N) insert may be identified by the absence of the marker gene function. In the third approach, recombinant expression vectors may be identified by assaying the Zn-peptidase (Aminopeptidase N) product expressed by the recombinant. Such assays may be based, for example, on the physical or functional properties of the Zn-peptidase (Aminopeptidase N) protein in *in vitro* assay systems *(e.g.,* binding with anti-Zn-peptidase (Aminopeptidase N) antibody or the Zn-peptidase (Aminopeptidase N) receptor).

Once a particular recombinant DNA molecule is identified and isolated, several methods known within the art may be utilized for propagation. Once a suitable host system and growth conditions are established, recombinant expression vectors may be propagated and prepared in quantity. As previously explained, the expression vectors which may be used include, but are not limited to, the following vectors or their derivatives: (*i*) human or animal viruses (*e.g*., vaccinia virus or adenovirus); (*ii*) insect viruses (*e.g*., baculovirus); (*iii*) yeast vectors; (*iv*) bacteriophage vectors (*e.g*., lambda); (*v*) plasmid and cosmid DNA vectors and the like.

In a further embodiment, a host cell strain may be chosen which modulates the expression of the inserted sequences, or modifies and processes the gene product in the specific manner desired. Expression from certain promoters may be elevated in the presence of certain inducers; thus, expression of the genetically engineered Zn-peptidase (Aminopeptidase N) protein may be controlled. Furthermore, different types of host cells possess characteristic and specific mechanisms for the translational and post-translational processing and modification (*e.g*., glycosylation, phosphorylation of proteins and the like). Appropriate cell lines or host systems may be chosen to ensure the desired modification and processing of the foreign protein expressed. For example, expression in a bacterial system may be used to produce a non-glycosylated core protein product; whereas expression in yeast will produce a glycosylated product. Expression in mammalian cells may be used to ensure "native" glycosylation of a heterologous protein. Furthermore, different vector/host expression systems may effect these processing mechanisms to varying extents.

In other specific embodiments of the present invention, the Zn-peptidase (Aminopeptidase N) protein (or derivative, fragment or analog) may be expressed as a fusion, or chimeric protein product (*i.e.*, comprising the protein, fragment, analog, or derivative joined via a peptide bond to a heterologous protein sequence of a different protein). Such a chimeric product may be produced by ligating the appropriate nucleic acid sequences encoding the desired amino acid sequences to one another by methods (in the proper coding frame), and expressing the chimeric product by methods which are well-known within the art. In an alternate embodiment, a chimeric product is generated by protein synthetic techniques (*e.g*., by use of a peptide synthesizer).

The Zn-peptidase (Aminopeptidase N) protein may also be isolated and purified by standard methods including chromatography (*e.g*., ion exchange, affinity, and partition chromatography), centrifugation, differential solubility or by any other standard technique for the purification of proteins known within the art. The functional properties of the isolated proteins may then be ascertained and evaluated by use of any suitable assay. Alternatively, the protein of interest may be synthesized by the numerous chemical methods known within the art (see *e*.*g*., Hunkapiller, *et al*., 1984. *Nature* 310:105-111).

In another alternate embodiment of the present invention, native Zn-peptidase (Aminpeptidase N) proteins may be purified from natural sources utilizing standard methods such as those described *supra* (*e.g*., immunoaffinity purification).

### (2) Treatment of Renal Disease

The present invention discloses uses of Zn-peptidase (Aminopeptidase N) in the manufacture of medicament for treating and preventing renal diseases (primarily renal failure and, preferably, acute renal failure) by the administration of one or more therapeutic compounds (hereinafter designated a "Therapeutic") of the present invention. The "Therapeutics" of the present invention include Zn-peptidase (Aminopeptidase N) proteins as well as the nucleic acid sequences (*i.e*., the genes) encoding the proteins (and derivatives, fragments and analogs thereof).

In the practice of the present invention, the subject to which the Therapeutic is to be administered is preferably an animal, such as including, but not limited to, cows, pigs, horses, chickens, cats, dogs, etc. and is more preferably a mammal. In the most preferred embodiment of the present invention, the subject is a human. Generally, the administration of the products of a species origin or species reactivity (in the case of antibodies) which is derived from the same species as that of the subject, is a preferred embodiment. Thus, in a preferred embodiment, a human Zn-peptidase (Aminopeptidase N) protein (or derivative, fragment or analog thereof) or a nucleic acid (or derivative, fragment or analog thereof, including anti-sense nucleic acid sequences thereof) are therapeutically or prophylactically to be administered to a human patient.

In a preferred embodiment, Therapeutics of the present invention are to be administered therapeutically, and preferably, prophylactically, to patients who are suffering from, or who are in danger of suffering from, renal failure or a renal disease, preferably, acute renal failure.

Therapeutics of the present invention may be administered either alone or in combination with other therapies (*e.g*., pharmaceutical compositions which are effective in the treatment or prevention of renal impairment). Therapeutics may also be concomitantly administered with drugs which treat or ameliorate the effect of certain risk factors, including, but not limited to, therapeutics which reduce cholesterol levels, treat obesity, treatment insulin-dependent and non-insulin-dependent diabetes mellitus (IDDM, NIDDM)) and the like. In a preferred embodiment of the present invention, a Therapeutic is to be administered with one or more anti-hypertensive drugs, including, but not limited to: (*i*) sympatholytics (*e.g*., propranolol, atenolol, nadolol, labetalol, prazosin, terazosin, doxazosin, clonidine, gugeneacine, methyldopa, reserpine, etc.); (*ii*) angiotensin inhibitors (*e.g*., benazepril, captopril, enalapril, losartan); (*iii*) calcium channel blockers *(e.g.,* diltiazem, felodipine, isradipine, nifedipine, verapamil); (*iv*) diuretics (*e.g*., thiazides - such as bendioflumethiazide, benzthiazide and hydrocholorothiazide; loop diuretics - such as bumetanide, ethacrynic acid, furosemide, and torsemide; potassium-sparing diuretics - such as amiloride, spironolactone and triametrene and various other types of diuretics and vasodilators - such as hydralazine and minoxidil).

It should also be noted that it is within the skill of those within the art to monitor and adjust the treatment or prophylactic regimen for the treatment or prevention of renal disease, while concomitantly treating or preventing other, potentially associated diseases or disorders (*e.g*., hypertension).

### (A) Gene Therapy

In a specific embodiment of the present invention, nucleic acids comprising a sequence encoding a Zn-peptidase (Aminopeptidase N) (or derivative, fragment or analog thereof) or a Zn-peptidase (Aminopeptidase N) anti-sense nucleic acid, are to be administered utilizing gene therapy methods. Gene therapy refers to a therapy which is performed by the administration of a specific nucleic acid (or derivative, fragment or analog) or an anti-sense nucleic acid, to a subject in need of such treatment. In the embodiment of the present invention, the nucleic acid produces its encoded protein or an anti-sense nucleic acid that mediates a therapeutic effect.

Any of the methods for gene therapy known within the art may be utilized in the practice of the present invention. For general reviews of the methods of gene therapy see *e.g.*, Goldspiel, *et al*., 1993. *Clin. Pharmacy* 12:488-505; Wu & Wu, 1991. *Biotherapy* 3: 87-95; Mulligan, 1993. *Science* 260:926-932; .Wu & Wu, 1991. *Biotherapy* 3:87-95; Tolstoshev, 1993. *Ann. Rev. Pharmacol. Toxicol*. 32:573-596; Morgan & Anderson, 1993. *Ann. Rev. Biochem*. 62:191-217 and Morgan & Anderson, 1993. *TIBTECH* 11:155-215.

In a preferred aspect, the Therapeutic comprises a Zn-peptidase (Aminopeptidase N) nucleic acid which is part of an expression vector that expresses a Zn-peptidase (Aminopeptidase N) protein (or derivative, fragment, analog or homolog thereof), a chimeric protein, preferably comprising a Zn-peptidase (Aminopeptidase N) protein (or derivative, fragment, analog or homolog thereof) or a Zn-peptidase (Aminopeptidase N) anti-sense nucleic acid thereof, within a suitable host. In a specific embodiment, such a nucleic acid possesses a promoter which is operably-linked to the Zn-peptidase (Aminopeptidase N) coding region, or to a sequence encoding a Zn-peptidase (Aminopeptidse N) anti-sense nucleic acid; wherein said promoter is inducible or constitutive and, optionally, tissue-specific. In another particular embodiment, a nucleic acid molecule is utilized in which the Zn-peptidase (Aminopeptidase N) coding sequence (and any other desired sequence) is flanked by regions which promote homologous recombination at a desired site within the genome, thus providing for intra-chromosomal expression of the Zn-peptidase (Aminopeptidase N) nucleic acid. See *e.g*., Koller & Smithies, 1989. *Proc. Natl. Acad. Sci. USA* 86:8932-8935; Zijlstra, *et al*., 1989. *Nature* 342:435-438.

In a preferred embodiment of the present invention, the Therapeutic comprises a Zn-peptidase (Aminopeptidase N) nucleic acid which is part of an expression vector that expresses the Zn-peptidase (Aminopeptidase N) protein (or derivatives, fragments, analogs or chimeric proteins thereof) within a suitable host. In particular, such a nucleic acid possesses a promoter operably-linked to the Zn-peptidase (Aminopeptidase N) coding region(s) or, less preferably, a separate promoter operably-linked to the Zn-peptidase (Aminopeptidase N) protein-coding region, wherein said promoter is inducible or constitutive and, optionally, tissue-specific.

Delivery of the nucleic acid into a patient may be either direct, in which case the patient is directly exposed to the nucleic acid or nucleic acid-carrying vector or indirect, in which case, cells are first transformed with the nucleic acid *in vitro,* then transplanted into the patient. These two approaches are known, respectively, as *in vivo* or *ex vivo* gene therapy. In a specific embodiment, the nucleic acid is to be directly administered *in vivo,* where it is expressed to produce the encoded product. This may be accomplished by any of numerous methods known in the art including, but not limited to, constructing it as part of an appropriate nucleic acid expression vector and administering it so that it becomes intracellular by: (*i*) infection using a defective or attenuated retroviral or other viral vector (see *e.g*., U.S. Patent No. 4,980,286); (*ii*) direct injection of naked DNA; (*iii*) use of microparticle bombardment (*e.g*., a gene gun - Biolistic, DuPont); (*iv*) coating with lipids or cell-surface receptors or transfecting agents, encapsulation in liposomes, microparticles, or microcapsules; (*v*) by administering it in linkage to a peptide which is known to enter the nucleus; (*vi*) administering it in linkage to a ligand subject to receptor-mediated endocytosis (see *e.g*., Wu & Wu, 1987. *J. Biol. Chem.* 262:4429-4432) which can be used to target cell types specifically-expressing the receptors and the like.

In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid may be targeted *in vivo* for cell specific uptake and expression, by targeting a specific receptor. See *e.g*., PCT Publications WO 93/14188; WO 93/20221. Alternatively, the nucleic acid may be introduced intracellularly and incorporated within host cell DNA for expression, by homologous recombination. See *e.g*., Koller & Smithies, 1989. *Proc. Natl. Acad. Sci. USA* 86:8932-8935; Zijlstra, *et al*., 1989. *Nature* 342:435-438.

In a one embodiment of the present invention, a viral vector that contains the Zn-peptidase (Aminopeptidase N) nucleic acid or, alternately, codes for Zn-peptidase (Aminopeptidase N) anti-sense nucleic acid, may be utilized. For example, a retroviral vector may be used (see *e.g*., Miller, et al., 1993. *Meth*. *Enzymol.* 217:581-599 (1993) which have been modified to delete retroviral sequences that are not necessary for packaging of the viral genome and integration into host cell DNA. Hence, the GENE SET nucleic acid to be utilized in gene therapy may be cloned into the vector, which facilitates delivery of the gene into a patient. A specific application of this technology may be found in Boesen, *et al*., 1994. *Biotherapy* 6:291-302, which describes the use of a retroviral vector to deliver the *mdrl* gene to hematopoietic stem cells in order to increase their resistivity to chemotherapy. Other references illustrating the use of retroviral vectors in gene therapy include: Clowes, *et al*., 1994. *J. Clin. Invest.* 93:644-651; Kiem, *et al*., 1994. *Blood* 83:1467-1473 and Salmons & Gunzberg, 1993. *Human Gene Therapy* 4:129-141.

Adenoviruses are other viral vectors that can be used in gene therapy. Adenoviruses are especially attractive vehicles for delivering genes to respiratory epithelia where the virus naturally infects to cause a mild disease. Other targets for adenovirus-based delivery systems are liver, the central nervous system, endothelial cells, and muscle. In addition, adenoviruses have the advantage of being capable of infecting non-dividing cells. See *e.g*., Kozarsky & Wilson, 1993. *Curr. Opin. Genet. Develop.* 3:499-503. Adeno-associated virus (AAV) has also been proposed for use in gene therapy. See *e.g*., Walsh, *et al*., 1993. *Proc. Soc. Exp. Biol. Med* 204:289-300.

Another approach to gene therapy involves transferring a gene into cells in tissue culture by such methods as electroporation, lipofection, calcium phosphate-mediated transfection, or viral infection. Generally, the method of transfer includes the transfer of a selectable marker to the cells which are then placed under selection to isolate those cells which have taken-up and are expressing the transferred gene and only those selected cells are then delivered to a patient. In this embodiment, the nucleic acid is introduced into a cell prior to administration *in vivo* of the resulting recombinant cell. Such introduction can be carried out by any method known in the art including, but not limited to, transfection, electroporation, microinjection, infection with a viral or bacteriophage vector containing the nucleic acid sequences, cell fusion, chromosome-mediated gene transfer, microcell-mediated gene transfer, spheroplast fusion, and the like (see *e.g*., Loeffler & Behr, 1993. *Meth*. *Enzymol*. 217:599-618; Cohen, *et al*., 1993. *Meth*. *Enzymol*. 217:618-644) and may be used in accordance with the present invention, provided that the necessary developmental and physiological functions of the recipient cells are not disrupted. The technique chosen should provide for the stable transfer of the nucleic acid to the cell, so that the nucleic acid is expressible by the cell and preferably heritable and expressible by its cell progeny. The resulting recombinant cells may be delivered to a patient by any of the methods well-known within the art.

With respect to the administration of the specific gene therapy agent, in a preferred embodiment of the present invention, epithelial cells are to be injected (*e.g*., subcutaneously). In another embodiment, recombinant skin cells may be applied as a skin graft onto the patient. Recombinant blood cells (*e.g*., hematopoietic stem or progenitor cells) are preferably administered intravenously. The amount of cells envisioned for use depends on the desired effect, patient state, etc., and may be determined by one skilled within the art. Cells into which a nucleic acid can be introduced for purposes of gene therapy encompass any desired, available cell type and include, but are not limited to: epithelial cells, endothelial cells, keratinocytes, fibroblasts, muscle cells, hepatocytes, blood cells such as T lymphocytes, B lymphocytes, monocytes, macrophages, neutrophils, eosinophils, megakaryocytes, granulocytes, various stem or progenitor cells, in particular hematopoietic stem or progenitor cells, other than derived from a human embryo (*e*.*g*., as obtained from bone marrow, umbilical cord blood, peripheral blood, fetal liver, etc). In a preferred embodiment of the present invention, the cell utilized for gene therapy is autologous to the patient.

In an embodiment in which recombinant cells are used in gene therapy, a Zn-peptidase (Aminopeptidase N) nucleic acid or nucleic acid encoding a Zn-peptidase (Aminopeptidase N) anti-sense nucleic acid is introduced into the cells such that it is expressible by the cells or their progeny, and the recombinant cells are then administered *in vivo* for therapeutic effect. In a specific embodiment, stem or progenitor cells, other than derived from a human embryo, are used. Any stem and/or progenitor cells which may be isolated and maintained *in vitro* may potentially be used in accordance with this embodiment of the present invention. Such stem cells include but are not limited to hematopoietic stem cells (HSC), stem cells of epithelial tissues such as the skin and the lining of the gut, embryonic heart muscle cells, liver stem cells (see *e.g*., PCT Publication WO 94/08598) and neural stem cells (see *e.g.,* Stemple & Anderson, 1992. *Cell* 71:973-985), other than derived from a human embryo.

Embryonic stem cells (ESCs) other than derived from a human embryo, or keratinocytes can be obtained from tissues such as the skin and the lining of the gut by known procedures. See *e.g*., Rheinwald, 1980. *Meth. Cell Bio*. 21:229-237. In stratified epithelial tissue such as the skin, renewal occurs by mitosis of stem cells within the germinal layer, the layer closest to the basal lamina. Stem cells within the lining of the gut provide for a rapid renewal rate of this tissue. ESCs other than derived from a human embryo or keratinocytes obtained from the skin or lining of the gut of a patient or donor can be grown in tissue culture. See *e.g.,* Pittelkow & Scott, 1986. *Mayo Clinic Proc.* 61:771-782. If the ESCs are provided by a donor, a method for suppression of host versus graft reactivity (*e.g*., irradiation, drug or antibody administration to promote moderate immunosuppression) may also be used. With respect to hematopoietic stem cells (HSC) other than derived from a human embryo, any technique which provides for the isolation, propagation, and maintenance *in vitro* of HSCs may be used in this embodiment of the present invention. Techniques by which this may be accomplished include, but are not limited to: (*i*) the isolation and establishment of HSC cultures from bone marrow cells isolated from the future host, or a donor or (*ii*) the use of previously established long-term HSC cultures, which may be allergenic or xenogeneic. Non-autologous HSC other than derived from a human embryo are used preferably in conjunction with a method of suppressing transplantation immune reactions of the future host/patient. In a specific embodiment of the present invention, human bone marrow cells can be obtained from the posterior iliac crest by needle aspiration. See *e.g.,* Kodo, *et al*., 1984. *J. Clin. Invest.* 73:1377-1384.

In a preferred embodiment of the present invention, the HSCs other than derived from a human embryo may be made highly enriched or in substantially pure form. This enrichment may be accomplished before, during or after long-term culturing, and may be performed by any techniques known in the art. Long-term cultures of bone marrow cells may be established and maintained by using, for example, modified Dexter cell culture techniques (see Dexter, *et al*., 1977. *J*. *Cell Physiol.* 91:335) or Witlock-Witte culture techniques (see Witlock & Witte, 1982. *Proc. Natl. Acad. Sci*. *USA* 79:3608-3612). In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

Additional methods which may be utilized, or adapted for utilization, for the delivery of a nucleic acid encoding a Zn-peptidase (Aminopeptidase N) protein (or functional derivative, fragment or analogs thereof) are described in Section 4, *infra*.

### (B) Antibodies

In one embodiment of the present invention, as discussed hereinabove, antibodies which possess the ability to bind Zn-peptidase (Aminopeptidase N) proteins (and derivatives, fragments or analogs thereof) may be utilized for the manufacture of a medicament to treat or prevent renal failure or acute disease or, preferably, acute renal failure. Anti-Zn-peptidase (Amniopeptidase N) antibodies may also be utilized in the methods useful for diagnostic, prognostic and screening disclosed by the present invention (*e.g*., as described in Section 3, *infra*). Such anti-Zn-peptidase (Aminopeptidase N) antibodies include, but are not limited to, polyclonal, monoclonal, chimeric, single-chain, F_{ab} fragments and an F_{ab} expression library. In a specific embodiment, antibodies specific for human Zn-peptidase (Aminopeptidase N) proteins are disclosed. In another specific embodiment, antibodies which possess the ability to reduce or inhibit Zn-peptidase (Aminopeptidase N) activity *in vitro* and/or *in vivo,* are also disclosed.

Various methods well-known within the art may be utilized for the production of polyclonal antibodies to a Zn-peptidase (Aminopeptidase N) protein or derivative or analog. In a particular embodiment, rabbit polyclonal antibodies specific for an epitope of a Zn-peptidase (Aminopeptidase N) protein or a nucleic acid encoding a Zn-peptidase (Aminopeptidase N) protein (and derivatives, fragments or analogs thereof) may be obtained. For the production of antibody, various host animals may be immunized by injection with the native Zn-peptidase (Aminopeptidase N) protein (or a synthetic version, derivative, fragment or analog thereof) including, but not limited to, rabbits, mice, rats, primates, etc. In addition, various adjuvants may be utilized to increase the immunological response, depending on the host species, and include, but are not limited to: Freund's (complete and incomplete); mineral gels (*e.g*., aluminum hydroxide); surface active substances (*e.g*., lysolecithin); pluronic polyols; polyanions; peptides; oil emulsions; keyhole limpet hemocyanins; dinitrophenol and potentially useful human adjuvants (*e.g*., *bacilli* Calmette-Guerin (BCG) and *corynebacterium parvum*).

For preparation of monoclonal antibodies which are specific for a Zn-peptidase (Aminpeptidase N) sequence (or derivative, fragment or analog thereof), any methodology which provides for the production of antibody molecules by continuous *in vitro* cell lines may be used. These methods include, but are not limited to: (*i*) the hybridoma technique (see *e.g*., Kohler & Milstein, 1975. *Nature* 256:495-497); (*ii*) the trioma technique (Cole, *et al*., 1985. In: *Monoclonal Antibodies and Cancer Therapy* (Alan R. Liss, Inc.); (*iii*) the human B-cell hybridoma technique (see *e.g*., Kozbor, *et al*., 1983. *Immunology Today* 4:72) and the EBV hybridoma technique to produce human monoclonal antibodies (see *e.g.,* Cole, *et al.*, 1985. In: *Monoclonal Antibodies and Cancer Therapy* (Alan R. Liss, Inc.). In an additional embodiment of the invention, monoclonal antibodies can be produced in germ-free animals utilizing a recently developed technology (see *e.g*., PCT Patent Publication U590/02545). Also within the scope of the present invention are the utilization of human antibodies which may be obtained through the use of human hybridomas (see *e.g.,* Cote, *et al*., 1983. *Proc. Natl. Acad. Sci*. *U.S.A*. 80:2026-2030) or, as previously discussed, by transforming human B cells with EBV virus *in vitro* (see *e.g.,* Cole, *et al*., 1985. In: *Monoclonal Antibodies and Cancer Therapy* (Alan R. Liss, Inc.).

In yet another embodiment, the techniques developed for the production of "chimeric antibodies" (see *e.g*., Morrison, *et al*., 1984. *Proc. Natl. Acad. Sci. U.S.A*. 81:6851-6855; Neuberger, *et al*., 1984. *Nature* 312:604-608; Takeda, *et al*., 1985. *Nature* 314:452-454) by splicing of the genes from a mouse antibody molecule specific for Zn-peptidase (Aminopeptidase N) together with genes from a human antibody molecule of appropriate biological activity may be utilized, and within the scope of the present invention. Alternately, non-human antibodies may be "humanized" by known methods (see *e.g*., U.S. Patent No. 5,225,539).

Also disclosed by the present invention are techniques for the production of single-chain antibodies (see *e.g*., U.S. Patent No. 4,946,778) may be adapted to produce Zn-peptidase (Aminopeptidase N) specific single-chain antibodies. An additional embodiment of the invention utilizes the techniques described for the construction of F_{ab} expression libraries (see *e.g.,* Huse, *et al*., 1989. *Science* 246:1275- 1281) to allow rapid and efficacious identification of monoclonal F_{ab} fragments with the desired specificity for Zn-peptidase (Aminopeptidase N) proteins (or derivatives or analogs thereof). In an alternative embodiment, antibody fragments which contain the idiotype of the molecule may be generated by known techniques. For example, such fragments include but are not limited to:
(*i*) the F(_{ab'})₂ fragment which may be produced by pepsin digestion of the antibody molecule;
(*ii*) the F_{ab'} fragments which may be generated by reducing the disulfide bridges of the F(_{ab'})₂ fragment; (*iii*) the Fab fragments which may be generated by treating the antibody molecule with papain and a reducing agent) and (*iv*) the Fᵥ fragments.

Screening for the desired antibody may be accomplished by any of the techniques which are well-known within the art including, but not limited to, enzyme-linked immunosorbent assay (ELISA). For example, to select antibodies which recognize a specific region (*e.g*., active site, transmembranal region and the like) of Zn-peptidase (Aminopeptidase N), the generated hybridomas may be may be examined for a product which binds to Zn-peptidase (Aminopeptidase N) fragment containing such a specific region. Similarly, for the selection of an antibody that may reduce or inhibit Zn-peptidase (Aminopeptidase N) activity, one may assay the antibody using any of the assays for Zn-peptidase (Aminopeptidase N) activity described in Section 6, *infra*.

### (C) Anti-Sense Nucleic Acids

In one embodiment of the present invention, Zn-peptidase (Aminopeptidase N) function may be reduced or inhibited through the use of Zn-peptidase (Aminopeptidase N) anti-sense nucleic acids, to treat or prevent renal failure or acute disease, preferably, acute renal failure. In a specific embodiment, nucleic acids of at least six nucleotides which are anti-sense to a gene or cDNA encoding a Zn-peptidase (Aminopeptidase N) protein (or a portion thereof) are used in a therapeutic or prophylactic manner. A Zn-peptidase (Aminopeptidase N) "anti-sense" nucleic acid, as used herein, refers to a nucleic acid which is capable of hybridizing to a portion of a Zn-peptidase (Aminopeptidase N) RNA (preferably mRNA) by virtue of some sequence complementarily. The anti-sense nucleic acid may be complementary to a coding and/or noncoding region of a Zn-peptidase (Aminopeptidase N) mRNA.

The Zn-peptidase (Aminopeptidase N) anti-sense nucleic acids of the present invention are comprised of at least six nucleotides and are, preferably, oligonucleotides (ranging from 6-150 nucleotides, or more preferably, 6 to 50 nucleotides). In specific embodiments, the oligonucleotide utilized in the practice of the present invention is at least 10 nucleotides, at least 15 nucleotides, at least 100 nucleotides, or at least 125 nucleotides. The oligonucleotides may be DNA, RNA or chimeric mixtures (or derivatives or modified versions thereof), and may be either single-stranded or double-stranded. In addition, the oligonucleotide may be modified at the base moiety, sugar moiety, or phosphate backbone. The oligonucleotide may also include other appending groups such as: (*i*) peptides facilitating transport across the cell membrane (see *e.g*., PCT Publication No. WO 88/09810) or blood-brain barrier (see *e.g.,* PCT Publication No. WO 89/10134); (*ii*) hybridization-triggered cleavage agents (see *e.g.,* Krol, *et al*., 1988. *BioTechniques* 6:958- 976) or (*iii*) intercalating agents (see *e.g.,* Zon, 1988. *Pharm. Res.* 5:539-549).

The Zn-peptidase (Aminopeptidase N) anti-sense nucleic acid is preferably an oligonucleotide, more preferably of single-stranded DNA. In another preferred embodiment, the oligonucleotide comprises a sequence anti-sense to a portion of human Zn-peptidase (Aminopeptidase N). The oligonucleotide may be modified at any position on its structure with substituents generally known within the art. In yet another embodiment, the oligonucleotide is an β-anomeric oligonucleotide which forms specific double-stranded hybrids with complementary RNA in which (contrary to the usual β-units) the strands run parallel to each other. See *e*.*g*., Gautier, *et al*., 1987. *Nucl. Acids Res.* 15:6625-6641. The oligonucleotide may also be conjugated to another molecule (*e*.*g*., a peptide, hybridization triggered cross-linking agent, transport agent, hybridization-triggered cleavage agent, etc.).

The anti-sense oligonucleotides of the invention may be synthesized by standard methods known in the art, for example, by use of an automated DNA synthesizer (such as are commercially available from Biosearch, Applied Biosystems, and the like). As examples, but not of limitations, phosphorothioate oligonucleotides may be synthesized by the method of Stein, *et al*. (1988. *Nucl. Acids Res.* 16:3209) and methylphosphonate oligonucleotides may be prepared by use of controlled pore glass polymer supports (see *e.g.,* Sarin, *et al*., 1988. *Proc. Natl. Acad. Sci. U.S.A*. 85:7448-7451).

In a specific embodiment of the present invention, the Zn-peptidase (Aminopeptidase N) anti-sense oligonucleotide comprises catalytic RNA, or a ribozyme (see *e.g.,* PCT Publication WO 90/11364; Sarver, *et al*., 1990. *Science* 247:1222-1225). In another embodiment, the oligonucleotide is a 2'-0-methylribonucleotide (see *e.g.,* Inoue, *et al*., 1987. *Nucl. Acids Res.* 15:6131-6148), or a chimeric RNA-DNA analogue (see *e.g.,* Inoue, *et al*., 1987. *FEBS Lett.* 215:327-330).

In an alternative embodiment of the present invention, the Zn-peptidase (Aminopeptidase N) anti-sense nucleic acid of the invention is produced intracellularly by transcription from an exogenous sequence. For example, a vector may be introduced *in vivo* such that it is taken up by a cell, within which cell the vector (or a portion thereof) is transcribed, resulting in the production of an anti-sense nucleic acid (RNA) of the invention. Such a vector would contain a sequence encoding the Zn-peptidase (Aminopeptidase N) anti-sense nucleic acid and may either remain episomal or become chromosomally integrated, so long as it is capable of being transcribed to produce the desired anti-sense RNA. Such vectors may be constructed by recombinant DNA technology methods standard within the art and may include, but not be limited to, plasmid, viral, or like vectors, which are used for replication and expression in mammalian cells. Expression of the sequence encoding the Zn-peptidase (Aminopeptidase N) anti-sense RNA may be by any promoter known within the art to function within mammalian or, preferably, human, cells. Such promoters may be inducible or constitutive and include, but are not limited to: (*i*) the SV40 early promoter region (see *e.g.,* Bernoist & Chambon, 1981. *Nature* 290:304-310); (*ii*) the promoter contained in the 3'-terminus long terminal repeat of Rous sarcoma virus (see *e.g.,* Yamamoto, *et al*., 1980. *Cell* 22:787-797);
*(iii)* the Herpesvirus thymidine kinase promoter (see *e.g*., Wagner, *et al*., 1981. *Proc. Natl. Acad. Sci. U.S.A*. 78:1441-1445) and (*iv*) the regulatory sequences of the metallothionein gene (see *e.g*., Brinster, *et al*., 1982. *Nature* 296:39-42).

The anti-sense nucleic acids of the present invention comprise a sequence complementary to at least a portion of an RNA transcript of a Zn-peptidase (Aminopeptidase N) gene, preferably a human Zn-peptidase (Aminopeptidase N) gene. However, absolute complementarily, although preferred, is not required. The term "a sequence complementary to at least a portion of an RNA," as utilized herein, is defined as a sequence possessing sufficient complementarily to enable it to hybridize with the RNA, resulting in the formation of a stable duplex; in the case of double-stranded Zn-peptidase (Aminopeptidase N) anti-sense nucleic acids, a single strand of the duplex DNA may thus be tested, or triplex formation may be assayed. The ability of the anti-sense nucleic acid to hybridize will depend upon both the degree of complementarily and the length of the anti-sense nucleic acid. Generally, the longer the hybridizing nucleic acid, the more base mismatches with a Zn-peptidase (Aminopeptidase N) RNA it may contain and still form a stable duplex (or triplex, as the case may be). One skilled within the art may ascertain a tolerable degree of mismatch by use of standard procedures to determine the melting point of the hybridized complex.

The present invention further provides pharmaceutical compositions comprising an effective amount of the Zn-peptidase (Aminopeptidase N) anti-sense nucleic acids of the invention in a pharmaceutically acceptable carrier, as described *infra*. In a specific embodiment, pharmaceutical compositions comprising Zn-peptidase (Aminopeptidase N) anti-sense nucleic acids may be administered via liposomes, microparticles, or microcapsules. In alternate embodiments, it may be useful to use such compositions to achieve sustained release of the Zn-peptidase (Aminopeptidase N) anti-sense nucleic acids. Additional methods that may be adapted for use in the delivery of a Zn-peptidase (Aminopeptidase N) anti-sense nucleic acid of the present invention will be disclosed in Section 5, *infra*.

The amount of Zn-peptidase (Aminopeptidase N) anti-sense nucleic acid which will be effective in the treatment or prevention of acute disease will be dependant upon the nature of the disease, and may be determined by standard clinical techniques. Where possible, it is desirable to determine the anti-sense cytotoxicity in cells *in vitro,* and then in useful animal model systems, prior to testing and *in vivo* use in humans.

### (3) Methods useful for Diagnosis, Prognosis and Screening

The present invention also discloses methods useful for diagnosis, prognosis and screening for renal failure or acute disease or, preferably, acute renal failure in individuals who include, but are not limited to, those subjects having renal disease or renal failure, having previously suffered an cerebrovascular event or exhibit one or more "risk factors" for renal failure or one or more conditions associated with renal failure.

In one embodiment of the present invention, anti-Zn-peptidase (Aminopeptidase N)-antibodies are used to detect and quantitate Zn-peptidase (Aminopeptidase N) levels in one or more tissues (*e.g*., blood) of a subject in immunoassays. In particular, such an immunoassay is performed by use of a method comprising contacting a sample derived from a patient with an anti-Zn-peptidase (Aminopeptidase N) antibody under conditions such that immunospecific-binding may occur, and subsequently detecting or measuring the amount of any immunospecific binding by the antibody. The particular amino acid deletion, insertion or substitution in the Zn-peptidase (Aminopeptidase N) may change the epitope recognized by a specific anti-(wild-type) Zn-peptidase (Aminopeptidase N) antibody such that antibody binds the Zn-peptidase (Aminopeptidase N) to a lesser extent or not at all. Additionally, antibodies may be produced (*e.g*., as described in Section 2(B), *supra)* against the Zn-peptidase (Aminopeptidase N) protein (or portion thereof) which possess the ability to immunospecifically-bind to the particular Zn-peptidase (Aminopeptidase N) protein, but not the wild type Zn-peptidase (Aminopeptidase N) protein (as determined by the *in vitro* immunoassay methods described below). These specific anti-Zn-peptidase (Aminopeptidase N) antibodies may be used to detect the presence of, for example, Zn-peptidase (Aminopeptidase N) proteins by measuring the immunospecific-binding by the anti-Zn-peptidase (Aminopeptidase N) protein antibodies and, optionally, lack of immunospecific binding by the anti-(wild-type) Zn-peptidase (Aminopeptidase N) protein antibodies. Additionally, Zn-peptidase (Aminopeptidase N) protein possessing deletion or insertion mutations may be detected by either an increase or decrease in protein size by, for example, but not limited to, Western blot analysis using an anti-Zn-peptidase (Aminopeptidase N) protein antibody that recognizes Zn-peptidase (Aminopeptidase N).

Immunoassay methods of the present invention which may be used include, but are not limited to, competitive and non-competitive assay systems using techniques such as Western blots, radioimmunoassay (RIA), enzyme linked immunosorbent assay(ELISA), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, and the like.

Kits for diagnostic or screening use are also provided by the present invention which comprise, in one or more containers, an anti-Zn-peptidase (Aminopeptidase N) antibody and (optionally) a labeled binding partner to the antibody. Alternatively, the anti-Zn-peptidase (Aminopeptidase N) antibody may be labeled with a detectable marker (*e.g.*, a chemiluminescent, enzymatic, fluorescent, or radioactive moiety). A kit is also provided which comprises, in one or more containers, a nucleic acid probe capable of hybridizing to Zn-peptidase (Aminopeptidase N) RNA or, preferably, capable of specifically hybridizing to a Zn-peptidase (Aminopeptidase N) gene. In a specific embodiment, a kit may comprise, in one or more containers, a pair of oligonucleotide primers (*e*.*g*., each in the size range of 6-30 nucleotides) which are capable of priming amplification of at least a portion of a Zn-peptidase (Aminopeptidase N) nucleic acid by: (*i*) polymerase chain reaction (PCR; see *e.g*., Innis, *et al*., 1990. *PCR Protocols* (Academic Press, Inc., San Diego, CA); (*ii*) ligase chain reaction; (*iii*) use of Qβ replicase; (*iv*) cyclic probe reaction, or other methods known in the art, under the appropriate reaction conditions. A kit may, optionally, further comprise, in one or more containers, a predetermined amount of a purified Zn-peptidase (Aminopeptidase N) protein or nucleic acid (*e.g*., for use as a standard or control).

### (4) Assays for the GENE SET Proteins or Modulators of the GENE SET Proteins

### and Nucleic Acids

A variety of methods are available within the art for use in assaying the activity of the GENE SET proteins (and derivatives, analogs, fragments and homologs thereof) and nucleic acids encoding the GENE SET proteins (and derivatives, analogs and fragments thereof). Methods are also available for the screening of putative GENE SET modulators (e.g., GENE SET protein agonists, antagonists and inhibitors). Such modulators of GENE SET protein activity include, but are not limited to, GENE SET anti-sense nucleic acids, anti-GENE SET antibodies, and competitive inhibitors of GENE SET for binding to the GENE SET protein receptor.

*In vitro* methods for assaying GENE SET proteins (and derivatives, fragments, homologs and analogs thereof), the nucleic acids encoding these GENE SET proteins, and putative modulators of GENE SET proteins (*e.g*., agonists, antagonists or inhibitors of GENE SET protein activity) include, but are not limited to: (i) GENE SET receptor binding assays (see *e.g*., Vesely, *et al*., 1992. *Renal Phys. Biochem.* 15:23-32; Iwashina, *et al*. 1994. *J. Biochem*. 115:563-567; Chang, *et al*., 1996. *Curr. Eye Res.* 15:137-143; (*ii*) measurement of changes in cGMP concentrations in cells possessing GENE SET receptors (see *e.g.,* Schulz, *et al*., 1989. *Cell* 58:1155-1162; Wedel, *et al*., 1997. *Proc. Natl. Acad. Sci. U.S.A.* 21:459-462 and (iii) changes in intracellular Ca²⁺ resulting from GENE SET receptor signaling in response to GENE SET binding (see *e.g*., Nascimento-Gomes, *et al*., 1995. *Brazil. J. of Med. Biol. Res.* 28:609-613. However, it should be noted that any measurement of GENE SET receptor activity elicited by GENE SET binding may be used to assay GENE SET activity *in vitro.*

The activity of the GENE SET proteins (and derivatives, fragments, analogs and homologs thereof), the nucleic acids encoding these GENE SET proteins (and derivatives, fragments, analogs and homologs thereof) and putative modulators of GENE SET activity may also be ascertained *in vivo.* By way of example, and not of limitation, the infusion of GENE SET proteins in humans causes significant increases in cGMP levels in both plasma and urine (see *e.g.,* Vesely, *et al*., 1995. *Am. J. Med. Sci.* 310:143-149; Vesely, *et al*., 1996. *Metabolism: Clin. & Exp*. 45:315-319). In addition, the administration of GENE SET proteins to humans also elicits significant diuresis and reduction in blood pressure (see *e.g.,* Vesely, *et al*., 1996. *Life Sciences* 59:243-254); similar effects have also been previously demonstrated in rodents (see *e.g*., Garcia, *et al*., 1989. *Hypertension* 13:567-574). Accordingly, the GENE SET proteins and nucleic acids (and derivatives, analogs, fragments and homologs thereof), as well as putative GENE SET modulators, may be assayed by administration of the test compound to a test animal (preferably a non-human test animal such as a rat or mouse), followed by the subsequent measurement of the one or more of the physiological parameters described above (*e.g.*, cGMP levels in urine and/or plasma, diuretic effect, decrease in blood pressure, and the like).

In a preferred embodiment of the present invention, rats derived from crosses with the fawn-hooded rat (FHR) may be used to assay for Zn-peptidase (Aminopeptidase N) protein, nucleic acid or modulator activity. For example, rats which possess the renal failure-predisposing locus on chromosome 1 with the concomitant lack of the renal failure-protective locus on chromosome 5 (which maps to the Zn-peptidase (Aminopeptidase N) gene) and, optionally, the other renal failure-protective locus on chromosome 4, may be used to screen for putative Zn-peptidase (Aminopeptidase N) modulators and antagonists. In a specific embodiment of the present invention, nucleic acids containing the nucleotide sequence encoding a Zn-peptidase (Aminopeptidase N) protein may be introduced into the rats possessing the chromosome 1 renal failure predisposing locus *but not* the renal failure protective loci. In accord, the Zn-peptidase (Aminopeptidase N) protein useful for treatment and prevention of renal failure would increase renal failure latency when either administered or transgenically-expressed in the renal failure prone rats lacking *both* the protective loci and fed a high salt diet to induce hypertension.

In another specific embodiment of the present invention, a putative modulator of Zn-peptidase (Aminopeptidase N) activity, or of latency or predisposition to renal failure, may be screened by:
(*i*) administering a putative modulator of Zn-peptidase (Aminopeptidase N) activity to an animal prone to renal failure and (*ii*) measuring one or more physiological parameters associated with Zn-peptidase (Aminopeptidase N) activity; wherein a change in one or more of the parameters, relative to an animal not administered the putative modulator, would be indicative of the putative modulator possessing the ability to modulate Zn-peptidase (Aminopeptidase N) activity or latency or predisposition to renal failure. In yet another specific embodiment, the animal prone to renal failure is fed a high salt diet. In a preferred embodiment of the present invention, the physiological parameter which may be measured is renal failure latency. Additionally, Zn-peptidase (Aminopeptidase N) modulators may be screened using a recombinant test non human animal which expresses a Zn-peptidase (Aminopeptidase N) transgene or expresses a Zn-peptidase (Aminopeptidase N) protein under the control of a promoter which is not the native (endogenous) Zn-peptidase (Aminopeptidase N) gene promoter, at an increased level relative to a wild-type test animal.

Another embodiment of the present invention discloses a methodology for screening a Zn-peptidase (Aminopeptidase N) protein, nucleic acid or modulator for the ability to alter Zn-peptidase (Aminopeptidase N) activity comprising: (*i*) administering the Zn-peptidase (Aminopeptidase N) protein, nucleic acid or modulator to a test animal prone to renal failure and (*ii*) measuring renal failure latency in the test animal in which renal failure latency is indicative of Zn-peptidase (Aminopeptidase N) activity. In a specific embodiment, a recombinant non human test animal which expresses a Zn-peptidase (Aminopeptidase N) transgene or expresses Zn-peptidsae (Aminopeptidase N) protein under the control of a promoter that is not the native Zn-peptidase (Aminopeptidase N) gene promoter at an increased level relative to a wild-type test animal is used to screen the Zn-peptidase (Aminopeptidase N) for a change in Zn-peptidase (Aminopeptidase N) activity.

In yet another specific embodiment, a method for screening for a modulator of Zn-peptidase (Aminopeptidase N) activity or of latency or predisposition to renal failure is provided which comprises measuring renal failure latency in a renal failure-prone animal which recombinantly expresses a putative modulator of Zn-peptidase (Aminopeptidase N) activity,
wherein a change in renal failure latency relative to an analogous renal failure-prone animal which does not recombinantly express the putative modulator is indicative of the putative modulator possessing the ability to modulate Zn-peptidase (Aminopeptidase N) activity or latency or predisposition to renal failure.

### (5) Pharmaceutical Compositions

The present invention discloses use of Zn-peptidase (Aminopeptidase N) in the manufacture of a medicament (Therapeutic) for treating or preventing renal disease. In a preferred embodiment, the Therapeutic (Zn-peptidase (Aminopeptidase N)) is substantially-purified. The subject is preferably an animal, including, but not limited to, animals such as cows, pigs, horses, chickens, cats, dogs, etc., and is preferably a mammal, and most preferably human.

Formulations and methods of administration which may be employed in the practice of the present invention when the Therapeutic comprises a nucleic acid are described in Section 2(A) and Section 2(C) *supra.* Additional appropriate formulations and routes of administration may be selected from among those described hereinbelow.

Various delivery systems are known and can be used to administer the Therapeutic of the invention including, but not limited to: encapsulation in liposomes, microparticles, microcapsules, recombinant cells capable of expressing the Therapeutic, receptor-mediated endocytosis (see *e.g.,* Wu & Wu, 1987. *J*. *Biol. Chem.* 262:4429-4432), construction of a Therapeutic nucleic acid as part of a retroviral or other vector, and the like. Methods of introduction include, but are not limited to: intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (*e.g*., oral mucosa, rectal and intestinal mucosa, etc.) and may be administered together with other biologically active agents. Administration may be either systemic or local. Furthermore, it may be desirable to introduce the pharmaceutical compositions of the invention into the central nervous system by any suitable route, including intraventricular and intrathecal injection, intraventricular injection may be facilitated by an intraventricular catheter, for example, attached to a reservoir (*e.g*., an Ommaya reservoir). Pulmonary administration may also be employed (*e.g*., by use of an inhaler or nebulizer, and formulation with an aerosolizing agent).

In another specific embodiment of the present invention, it may be desirable to administer the pharmaceutical compositions of the invention locally to the area in need of treatment, this may be achieved by, for example, and not by way of limitation, local infusion during surgery, topical application (*e.g*., in conjunction with a wound dressing after surgery, by injection, by means of a catheter, by means of a suppository, or by means of an implant, said implant being of a porous, non-porous, or gelatinous material, including membranes, such as sialastic membranes, or fibers). In one embodiment, administration can be by direct injection at the site (or former site) of a malignant tumor or neoplastic or pre-neoplastic tissue. In another embodiment, the Therapeutic may be delivered in a vesicle, in particular a liposome (see *e.g*., Treat, *et al*., In: *Liposomes in the Therapy of Infectious Disease and Cancer* (Liss, New York, NY).

In yet another embodiment, the Therapeutic may be delivered in a controlled release system. In one specific embodiment, a pump may be utilized. See *e.g.,* Sefton, 1987. *CRC Crit. Ref. Biomed. Eng.* 14:201. In another embodiment, polymeric materials can be used (see *e.g*., *Medical Applications of Controlled Release* 1984. (CRC Pres., Boca Raton, FL). In yet another embodiment, a controlled release system can be placed in proximity of the therapeutic target (*e*.*g*., the brain), thus requiring only a fraction of the systemic dose. In a specific embodiment where the Therapeutic is a nucleic acid encoding a protein Therapeutic, the nucleic acid may be administered *in vivo* to promote expression of its encoded protein, by constructing it as part of an appropriate nucleic acid expression vector and administering it so that it becomes intracellular, *(e.g.,* by use of a retroviral vector; see U.S. Patent No. 4,980,286), or by direct injection; or by use of microparticle bombardment (*e.g*., a gene gun, Biolistic, DuPont), or coating with lipids or cell-surface receptors or transfecting agents; or by administering it in linkage to a homeobox-like peptide which is known to enter the nucleus (see *e.g*., Joliot, *et al*., 1991. *Proc. Natl. Acad. Sci. USA* 88:1864-1868), and the like. Alternatively, a nucleic acid Therapeutic can be introduced intracellularly and incorporated within host cell DNA for expression, by homologous recombination.

The present invention also provides pharmaceutical compositions. Such compositions comprise a therapeutically effective amount of a Zn-peptidase (Aminopeptidase N), and a pharmaceutically acceptable carrier. In a specific embodiment, the term "pharmaceutically acceptable," as used herein, means approved by a regulatory agency of the Federal or a state government or listed in the U.S. Pharmacopeia or other generally recognized pharmacopeia for use in animals, and more particularly, in humans. The term "carrier," as used herein, refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "*Remington's Pharmaceutical Sciences"* by E.W. Martin.

Such compositions will contain a therapeutically effective amount of the Therapeutic (preferably in a substantially purified form) together with a suitable amount of carrier so as to provide the form for proper administration to the patient. The formulation should suit the mode of administration. In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic (*e.g.*, lignocaine) to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration. The Therapeutic of the invention can be formulated as neutral or salt forms.

The amount of the Therapeutic of the present invention which will be effective in the treatment of a particular disorder or condition will depend on the nature of the disorder or condition, and may be determined in a quantitative manner by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 µg of active compound/kg of patient body weight. Suitable dosage ranges for intranasal administration are generally about 0.01 pg/kg body weight to 1 mg/kg body weight. Effective doses may be extrapolated from dose-response curves derived from *in vitro* or animal model test systems. Suppositories generally contain active ingredient in the range of 0.5% to 10% by weight; whereas oral formulations preferably contain 10% to 95% active ingredient.

The present invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### (6) Animal Models

The present invention also provides non-human animal models. In a specific embodiment, non-human animal models for renal failure or ischemic disease, specifically acute renal failure are provided. In one embodiment, FHRs may be bred with normal or non-renal failure-prone rats not possessing a Zn-peptidase (Aminopeptidase N) allele. Rats may then be selected which possess the chromosome 1 locus for renal failure predisposition but do not have the chromosome 5 locus (*i.e*., possess a wild-type Zn-peptidase (Aminopeptidase N) locus) or, optionally, the chromosome 4 locus, demonstrated to be protective for renal failure in the FHR strain. Such animals may be used to test for Zn-peptidase (Aminopeptidase N) proteins with reduced activity or for Zn-peptidase (Aminopeptidase N) antagonists as described in Section 6, *supra*.

In another embodiment of the present invention, non-human transgenic animals may be bred or produced through molecular-biological means, which over-express or under-express the Zn-peptidase (Aminopeptidase N) gene (*e.g*., by introducing a member or members of the Zn-peptidase (Aminopeptidase N) gene under the control of a heterologous promoter or a promoter which facilitates the expression of Zn-peptidase (Aminopeptidase N) protein and/or nucleic acids in tissues which do not normally express Zn-peptidase (Aminopeptidase N). Additionally, "knockout" mice may be initially produced by promoting homologous recombination between the Zn-peptidase (Aminopeptidase N) gene in its chromosome and an exogenous Zn-peptidase (Aminopeptidase N) gene that has been rendered biologically inactive, preferably by insertion of a heterologous sequence (*e.g.*, an antibiotic resistance gene) or by non-homologous recombination.

In a preferred embodiment of the present invention, introduction of heterologous DNA is carried out by transforming embryo-derived non-human stem (ES) cells with a vector containing the insertionally-inactivated Zn-peptidase (Aminopeptidase N) gene or a Zn-peptidase (Aminopeptidase N) gene which was under the control of a heterologous promoter, followed by injecting the ES cells into a blastocyst and implanting the blastocyst into a "foster mother" non-human animal. Accordingly, the resulting mice are chimeric animals ("knockout animal" or "transgenic animal") in which a Zn-peptidase (Aminopeptidase N) gene has been inactivated or overexpressed or misexpressed (see *e.g.,* Capecchi, 1989. *Science* 244:1288-1292). The chimeric non-human animal can then be bred to produce additional knockout or transgenic non-human animals. Such chimeric/transgenic animals include, but are not limited to, mice, hamsters, sheep, pigs, cattle, etc., and are, non-human mammals. Transgenic and knockout animals can also be made in *D. melanogaster*, *C. elegans*, and the like, by methods which are commonly-known within the art.

### (7) Specific Examples

The quantitative expression analysis (QEA®) methodology (see PCT Publication WO 97/15690, dated May 1, 1997 to Nandabalan, *et al*), which has now been registered as the trademark GeneCalling®, was utilized to identify and characterize genes which were, putatively, differentially-expressed within the FHR and IRL rodent strains (*i*.*e*., renal disease animal models) as compared to the ACI (control) rodent strain. It should be noted, however, that RNA reverse transcriptase polymerase chain reaction (RT-PCR) may also be, preferably, utilized to identify the differentially-expressed GENE SET genes/gene products in the practice of the present invention. Both the GeneCalling® and RT-PCR methods will be discussed in detail in the following sections.

Prior to utilization in the assays disclosed herein, the degree of proteinuria exhibited by both the FHR and IRL rodent strains, in comparison to the control ACI strain, is quantitatively determined. Both the FHR and IRL rodent strains exhibited marked proteinuria.

### (A) Experimental Methods and Materials of the Present Invention

### (I) Identification of GENE SET Genes by GeneCalling®

### (i) Tissue Dissection

Kidney tissue derived from the FHR, IRL and ACI (control) rodent strains were analyzed by the proprietary GeneCalling® methodology.

In brief, FHR, IRL and ACI rats were maintained on normal rat chow and water *ad libitum.* FHR and the control ACI rats were sacrificed at 1.5 and 7.5 months-of-age, whereas the IRL is sacrificed only at 1.5 months-of-age. While the 1.5 month rats appeared "normal" as a result of laboratory blood-work which is performed, the FHR by 7.5 months-of-age is demonstrated to have developed pronounced proteinuria. This finding is in agreement with previous experimental results which found that the median age of onset of proteinuria (and other renal-related disorders) in the FHR was approximately 4 months-of-age. Following the abnormal laboratory results indicating the onset of proteinuria, the animals were sacrificed and their kidneys removed and quick-frozen in liquid nitrogen immediately after dissection. The kidneys were stored at -70°C until utilized in the subsequent GeneCalling® protocols.

### (ii) Isolation of Total Cellular and Poly(A)⁺ RNA

Total cellular RNA was extracted from 5 mg of heart, liver, fat, kidney, or brain tissue by initially grinding the tissue into a fine powder in liquid nitrogen. The powdered tissue was then transferred to a tube containing 500 µl Triazol Reagent® (Life Technologies; Gaithersburg, MD) and was dispersed using a Polytron homogenizer (Brinkman Instruments; Westbury, NY). See *e.g*., Chomszynski, *et al*. 1987. *Annal. Biochem*. 162 156-159; Chomszynski, *et al*., 1993. *Biotechniques* 15:532-533, 536-537. The total cellular RNA fraction was then extracted with 50 µl BCP (1-bromo-3-chloropropane; Molecular Research; Cincinnati, OH) to facilitate phase separation. The extraction mixture was centrifuged for 15 minutes at 4°C at 12,000 x G, and the aqueous phase was removed and transferred to a fresh tube. The RNA was then precipitated with 0.5 volume of isopropanol per original volume of Triazol Reagent® used, and the sample was re-centrifuged at room temperature for 10 minutes at 12,000 x G. The supernatant was then discarded, the pellet washed with 70% ethanol and re-centrifuged at room temperature for 5 minutes at 12,000 x G. Finally the 70% ethanol was removed and the centrifuge tube was inverted and let stand to dry in this position. The resulting RNA pellet was re-suspended in 100 µl water (*i.e*., 1 µl/mg of original tissue weight) and heated to 55°C until completely dissolved. The final concentration of total cellular RNA was quantitated by fluorometry with OliGreen® (Molecular Probes; Eugene, OR). In addition, the quality of the total cellular RNA was determined by both spectrophotometry and formaldehyde agarose gel electrophoresis.

Poly(A)⁺ mRNA was prepared from 100 µg of total cellular RNA by use of affinity chromatography with oligo(dT) magnetic beads (PerSeptive; Cambridge, MA) or with the Dynabeads mRNA Direct Kit® (Dynal; Oslo, Norway) as directed by the manufacturer. The Poly(A)⁺ mRNA was harvested in a small volume of sterile water, and the final yield quantified by OD₂₆₀ measurement and fluorometry with OliGreen® (Molecular Probes; Eugene, OR). The Poly(A)⁺ mRNA was stored at -20°C for subsequent utilization in GeneCalling® protocols.

### (ii) cDNA Synthesis

The RNA samples were then treated with DNase to remove endogenous, contaminating DNA. 28 µl of 5X reverse transcriptase buffer (Life Technologies; Gaithersburg, MD), 10 µl 0.1 M DTT, 5 units RNAguard® (Pharmacia Biotech, Upsala, Sweden) per 100 mg tissue and 1 unit RNase-free DNase I® (Pharmacia Biotech) per 100 mg tissue, were added to the re-suspended RNA samples. The reaction mixture was then incubated at 37°C for 20 minutes. The total RNA concentration was quantified by measuring OD₂₆₀ of a 100-fold dilution and the samples stored at -20°C.

cDNA was synthesized from the Poly(A)⁺ RNA as follows: the Poly(A)⁺ RNA was mixed with 50 ng random hexanucleotides (50 ng/µl) in 10 µl of water. The mixture was heated to 70°C for 10 minutes, quick-chilled in ice-water slurry, and kept on ice for 1-2 minuets. The condensate was collected by centrifugation in a microfuge for 10 seconds. In an alternate embodiment of the present invention, 200 pmols oligo(dT)₂₅V (where V = A, C or G) was utilized in place of the random hexanucleotide primers.

The first strand synthesis was carried out by adding a reaction mixture consisting of the following reagents: 4 µl 5X first strand buffer (BRL; Grand Island NY), 2 µl 100 mM DTT, 1 µl 10 mM dNTP mix and 2 µl water to the primer-annealed RNA. The reaction mixtures were then incubated at 37°C for 2 minuets and 1 µl of Superscript II® reverse transcriptase (BRL) was added following the manufacturer's recommendation and the reactions were then incubated at 37°C for 1 hour.

To synthesize the second cDNA strand, the samples were placed on ice, 30 µl of 5X Second strand buffer, 90 µl of cold water, 3 µl of 10 mM dNTP, 1 µL (10 units) of *E. coli* DNA ligase (BRL), 4 µl (40 units) of *E. coli* DNA polymerase (BRL), and 1 µl (3.5 units) of *E. coli* RNase H (BRL) were added to the tubes, and the reactions were incubated for 2 hours at 16°C. The resulting cDNA was then incubated with 2 µl of T4 DNA polymerase (5 units) at 16°C for 5 minuets.

The resulting cDNA was dephosphorylated with Arctic Shrimp Alkaline Phosphatase ("SAP"; US Biochemicals; Cleveland, OH) by the addition of 20 µl 10X SAP buffer, 25 µl of water, and 5 µl (5 units) of SAP to the reaction mixtures which were then incubated at 37°C for 30 minuets.

The cDNA was extracted with phenol-chloroform (50:50 v/v), chloroform-isoamyl alcohol (99:1 v/v), and precipitated from the aqueous phase by the addition of NaOAc (pH 5.0) to a final concentration of 0.3 M, 20 µg glycogen and 2 volumes of ethanol. The reactions were incubated at -20°C for 10 minuets and the cDNA was collected by centrifugation at 14,000 x g for 10 minuets The supernatant was aspirated and the cDNA pellet washed with 75% ethanol, resuspended in TE, and the yield of cDNA was estimated using fluorometry with Picogreen® (Molecular Probes, Eugene OR).

### (iii) GeneCalling® Expression Analysis

For subsequent GeneCalling® expression analysis, 75 ng cDNA was transferred to a separate tube, resuspended in TE to a concentration 600 ng/ml and stored at -20°C.

GeneCalling® analysis was performed as disclosed in PCT Publication WO 97/15690, dated May 1, 1997 to Nandabalan, *et al*. By way of example, and not of limitation, an exemplar GeneCalling® analysis performed on the PC4 gene encoding IRPR (INF-β) will be disclosed herein. Restriction endonuclease (RE) digestion of the INF-β-encoding nucleic acid was performed with BsrFI and BglII restriction endonucleases. Adapter molecules for the subsequent GeneCalling® analysis were prepared from linker and primer oligonucleotides. For the "sticky" termini generated by the BsrFI restriction endonuclease, the linker oligonucleotide:
5'-GGCCCGAAGTACA-3' [SEQ ID NO:1] and the primer oligonucleotide:
5'-GGCCCGAAGTAC-3' [SEQ ID NO:2], were used. For the "sticky" termini generated by the BglII restriction endonuclease, the linker oligonucleotide: 5'-GGCCCAGCCACT-3' [SEQ ID NO:3] and the primer oligonucleotide: 5'-GGCCCAGCCAC-3' [SEQ ID NO:4] were used. One set of the primers was labeled with a FAM fluorescent label and the other set was labeled with a biotin moiety. The adapters were prepared by mixing the linker and primer oligonucleotides together in water at a concentration ratio of 1:20 (linker to primer) with the primer held at a total concentration of 50 pm/µl. The reaction mixture was incubated at 50°C for 10 minutes and then allowed to cool slowly to room temperature to anneal the linkers and primers. The adapters were stored at -20°C. It should be noted that other oligonucleotide linkers and primers would be required for use with the nucleic acid sequences encoding one of the other GENE SET proteins. By necessity, the sequences of these oligonucleotide molecules would be dependent upon the nucleic acid sequences of the specific GENE SET sequences, and would be readily ascertainable by individuals skilled within the art.

The GeneCalling® reactions were performed using an automated GeneCalling® procedure. Reactions were preformed in a standard 96-well thermal cycler format using a Beckman Biomek 2000® robot (Beckman; Sunnyvale, CA). The cDNA samples were analyzed in triplicate with BsrFI and BglII restriction enzymes. All steps were performed by the robot, including solution mixing (from user provided stock reagents) and temperature profile control.

The RE/ligase reaction contained the following components per reaction: 1 U each of BsrFI and BglII (New England Biolabs; Beverly, MA), 1 µl of each annealed adapter prepared as above (10 pm), 0.1 µl T₄ DNA ligase [1 Unit/µl] (Life Technologies, Gaithersburg, MD), 1 µl 10 mM ATP (Life Technologies), 5 ng of the prepared cDNA, 1.5 µl 10X NEB 2 buffer (New England Biolabs), 0.5 µl of 50 mM MgCl₂ and water to bring the total volume to 10 µl. The reactions were then transferred to thermal cycler.

The robot performed the RE/ligation reaction in a PTC-100® Thermal Cycler equipped with a mechanized lid (MJ Research; Watertown, MA) with the following temperature profile: 15 minutes at 37°C, ramp down 21°C in 5 minutes, 16°C for 30 minutes, 37°C for 10 minutes and 65°C for 10 minutes.

The PCR reaction mix contained the following components per reaction: 10 µl 5X E-Mg (300 mM Tris-HCl pH 9.0, 75 mM (NH₄)₂SO₄), 100 pm of BsrFI- and BglII-primers [SEQ ID NO:16 and NO:18, respectively], 1 µl 10 mM dNTP mix (Life Technologies), 2.5 Units of 50:1 dilution of KlenTaq polymerase (Life Technologies):PFU polymerase (Stratagene, La Jolla, CA), and water to being the total reaction volume to 35 µl per PCR reaction.

The PCR reaction was then heated to 72°C and 35 µl was transferred to each separate digestion/ligation reaction. The PTC-100® Thermal Cycler then performed the PCR reaction with a thermal profile of 72°C for 10 minutes, 15 cycles of 95°C for 30 seconds and 68°C for 1 minute, and then 72°C for 10 minutes, and finally holding the reactions at 4°C.

Prior to further analysis, the GeneCalling® products were subjected to a post-PCR clean up protocol as follows: MPG® streptavidin magnetic beads (CPG; Lincoln Park, NJ) were prepared (3 µl of beads for every 5 µl of GeneCalling® reaction product) by pre-washing the beads in 10 µl binding buffer (5 M NaCl, 10 mM TRIS, pH 8.0, 1 mM EDTA) per 5 µl original volume of GeneCalling® reaction product. 10 µl of washed beads was dispensed in a 96 well FALCON® TC plate for every GeneCalling® sample processed. GeneCalling® products were added to the beads, mixed well and incubated for 30 minutes at 50°C. The sample volume was made 100 µl with binding buffer, the plate placed on a 96 well magnetic particle concentrator, and the beads allowed to migrate for 5 minutes. The liquid was then removed, and 200 µl washing buffer (10 mM Tris, pH 7.4, 10 mM EDTA) added per well. The washing step was then repeated.

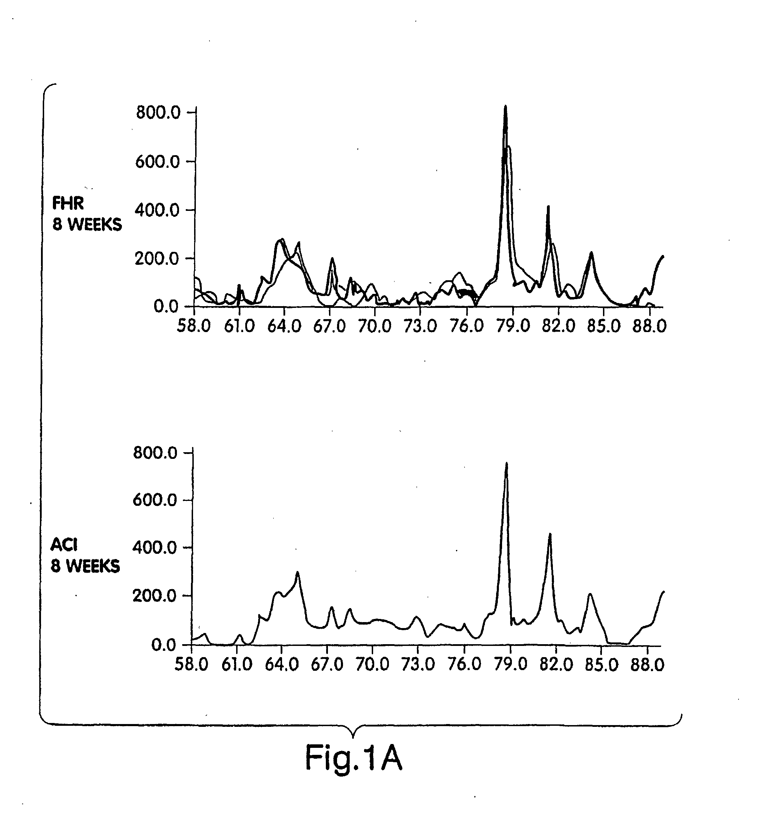

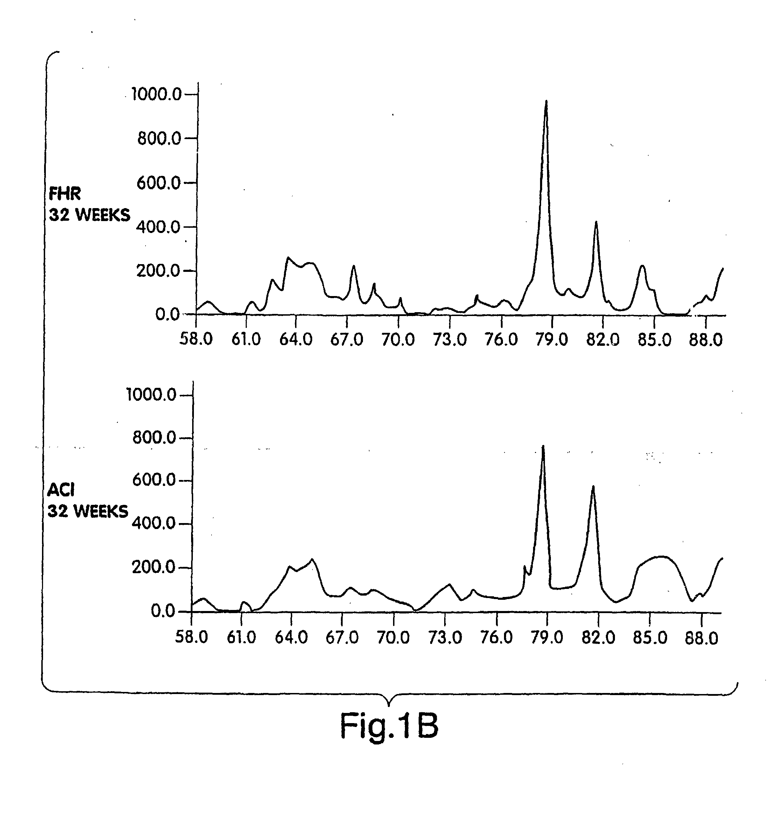

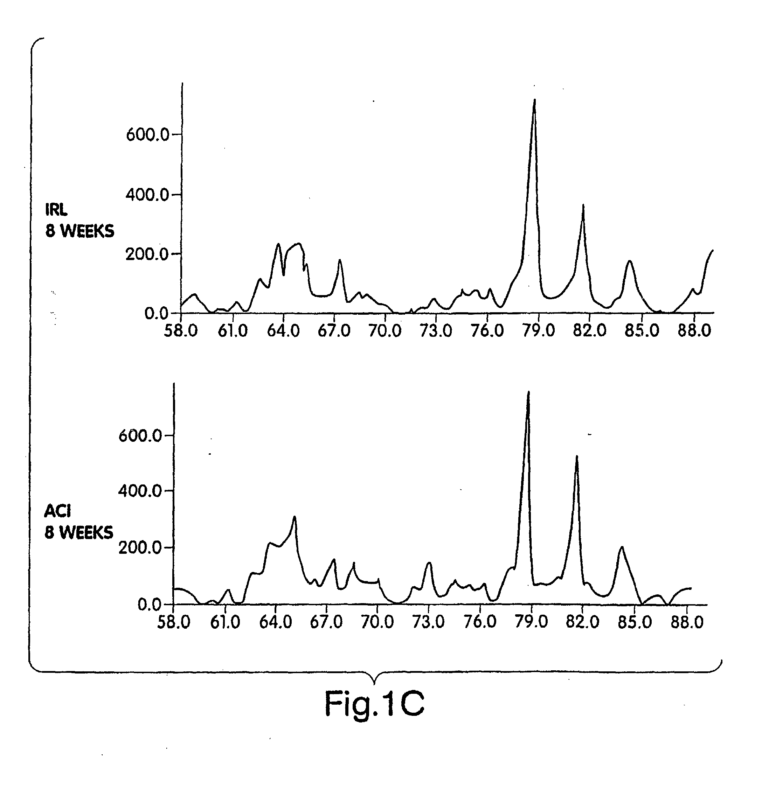

For analysis, the beads were resuspended in 5 µl loading buffer (80% deionized formamide, 20% 25 mM EDTA, pH 8.0, 50 mg/ml Blue Dextran) per 5 µl of beads, and the supernatant was then analyzed by electrophoresis on an ABI 377® (Applied Biosystems, Inc.) automated sequencer under denaturing conditions using the GeneSmay® computer software (ABI) for analysis. A GeneSmay 500® ROX ladder (diluted 1:10 in gel sample loading buffer) was utilized to a size control during the subsequent GeneCalling® analysis. The results obtained by the GeneCalling® methodology for the differential-expression of the PC4 gene encoding IRPR (INF-β) is illustrated in Figure 1.

"Oligonucleotide poisoning" was then performed to confirm that the putative differentially-expressed fragment were the genes which were originally predicted.

### (iv) Confirmation of the GeneCalling® Results by the Oligo-Poisoning™ Methodology

The present invention uses a positive confirmation methodology, known as Oligo-Poisoning™, that identifies nucleic acids containing putatively identified sequences predicted to generate observed GeneCalling® signals, that are actually present in the sample. This method confirms the presence of a specific, defined flanking nucleic acid subsequence which is adjacent to the "target" subsequence recognized by the probing means within a nucleic acid-containing sample. Importantly, Oligo-Poisoning™ is also equally applicable to confirming putative sequence identifications in any sample of nucleic acid fragments which possess a certain generic sequence structure or motif. This generic structure only limits fragments to have known terminal subsequences capable of acting as PCR primers.

Oligo-Poisoning™ proceeds by initially performing PCR amplification of, for example GeneCalling® reaction products, so as to produce detectable results for all nucleic acid fragments contained within the GeneCalling® reaction which *do not* possess the putatively identified subsequence. In the preferred embodiment of the present invention, this is achieved by adding a molar excess of a "poisoning" primer designed to amplify only those nucleic acid fragments having the putatively identified subsequence. The poisoning primer may, preferably, be unlabeled or it may be labeled so as to allow it to be differentiated from any other type of label utilized in the PCR amplification reaction. Following PCR amplification, the resulting reaction products are then separated by electrophoresis and the electrophoretic mobilities of the various fragments are examined. As those nucleic acid fragments containing the putatively identified subsequence which have undergone amplification will be, preferably, unlabeled, they will not generate a detectable signal.

Generally, the parameters of the PCR amplification reactions utilized in Oligo-Poisoning™ confirmation methodology are, preferably, similar or identical to those used in the generation of the initial GeneCalling® signals. This is especially advantageous in the case of application of Oligo-Poisoning™ to GeneCalling,® due to the fact that the "poisoned" signals may be readily compared to the initial GeneCalling® signals. Details of exemplary, preferred PCR protocols are described in following sections. In particular, for example, it is preferable that a hot start PCR method be used, and the preferable hot start method utilizing the wax layering technique will be subsequently described in further detail, *infra*.

In the practice of the wax laying technique of the present invention, PCR reaction vessels are set up by placing dNTPs and water in the lower portion of a reaction vessel; layering wax on top of this dNTP solution; and placing the remainder of the PCR reaction mix on top on the wax layer. As previously described, the wax used preferably melts rapidly at near but less than 72°C, the temperature preferred for the extension phase of the PCR amplification. During PCR amplification, the first thermal cycle begins with a denaturing temperature of approximately 96°C, which is adequate to melt the wax, cause mixing of the reagent compartments, and initiate amplification. The PCR thermal profile is performed, as described in the following section with a preferred stringent annealing temperature of at least approximately 57°C. Also, one primer of the pair of regular primers used in the PCR amplification may be biotinylated, thus allowing the utilization of the magnetic bead separation technique to facilitate the removal remove fragments from the input sample.

The last GeneCalling® step is separation according to length of the amplified fragments followed by detection the fragment lengths and end labels (if any). Lengths of the fragments excised from a cDNA sample typically span a range from a few tens of base pairs to perhaps 1000 bp. Any separation method with adequate length resolution, preferably at least to three bp in a 1000 base pair sequence, can be used. It is preferred to use gel electrophoresis in any adequate configuration known in the art. Gel electrophoresis is capable of resolving separate fragments which differ by three or more base pairs and, with knowledge of average fragment composition and with correction of composition induced mobility differences, of achieving a length precision down to 1 bp. A preferable electrophoresis apparatus is an ABI 377™ (Applied Biosystems, Inc.) automated sequencer using the Gene Scan™ software (ABI) for analysis. The electrophoresis can be done by suspending the reaction products in a loading buffer, which can be non-denaturing, in which the dsDNA remains hybridized and carries the labels (if any) of both primers. The buffer can also be denaturing, in which the dsDNA separates into single strands that typically are expected to migrate together (in the absence of large average differences in strand composition or significant strand secondary structure).

The length distribution is detected with various detection means. If no labels are used, means such as antigen (Ag) and antibody (Ab) staining and intercalating dyes can be used. Here, it can be advantageous to separate reaction products into classes, according to the previously described protocols, in order that each band can be unambiguously identified as to its target end subsequences. In the case of fluorochrome labels, since multiple fluorochrome labels can be typically be resolved from a single band in a gel, the products of one recognition reaction with several REs or other recognition means or of several separate recognition reactions can be analyzed in a single lane. However, where one band reveals signals from multiple fluorochrome labels, interpretation can be ambiguous: is such a band due to one fragment cut with multiple REs or to multiple fragments each cut by one RE. In this case, it can also be advantageous to separate reaction products into classes.

Oligo-Poisoning™ confirmation methodology is comprised of an unlabeled oligonucleotide possessing a nucleotide sequence which is capable of hybridizing to the GENE SET sequence(s) of interest was included in a PCR reaction using the GeneCalling® reaction products as substrate, thus preventing amplification with the labeled primers. Specifically, each of the oligonucleotide poisoning reaction mixtures contained: 1 µl of a 1:100 dilution of the GeneCalling® reaction products, 5 µl TB 2.0 (500 mM TRIS-HCl (pH 9.15), 160 mM (NH₄)₂SO₄, 20 mM MgCl₂, 2 µl 10 mM equimolar mixture of dNTPs, 0.2 µl each BsrFI and BglII primers
(100 pm/ml), 2 µl GENE SET "oligonucleotide poisoning" primer (1000 pm/ml), 1 µl 5 M betaine, 1 µl NEB 2 buffer (10 mM TRIS-HCl, 10 mM MgCl₂, 50 mM NaCl, 1 mM DTT (pH 7.9 at 25°C), 0.25 µl (25 U/µl) of a 16:1 dilution of KlenTaq:PFU and 38 µl water.

The following PCR amplification protocol was performed for a total of 13 cycles in a thermal cycler: 96°C for 30 seconds; 57°C for 1 minute; 72°C for 2 minutes. The amplified products were then held at 4°C for subsequent analysis utilizing automated sequencing apparatus as previously described, *supra*.

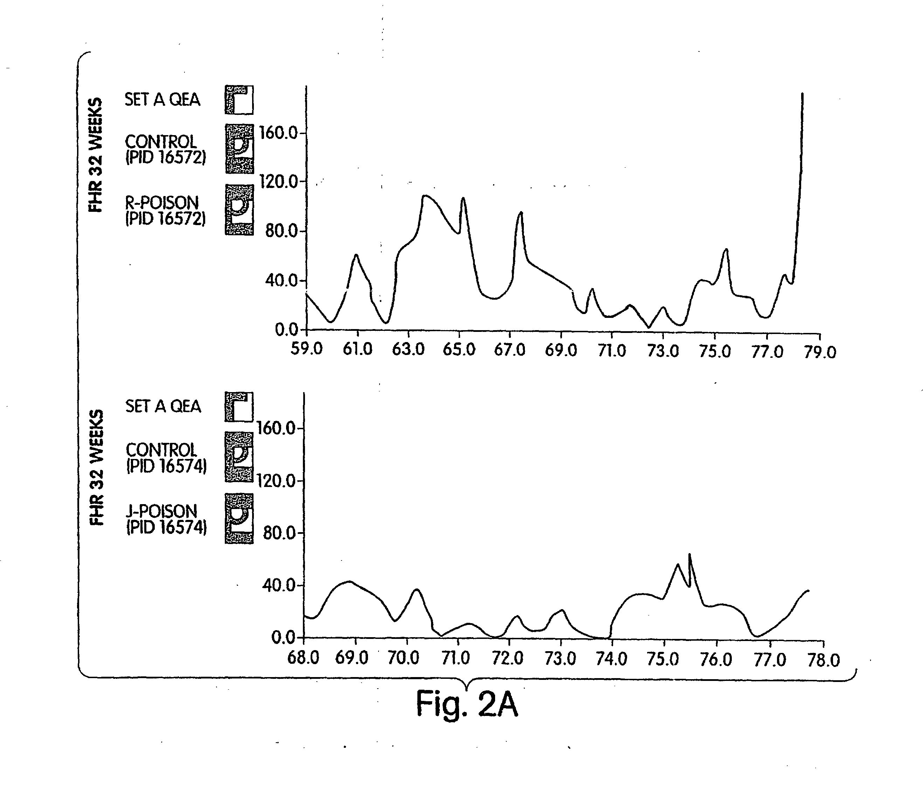

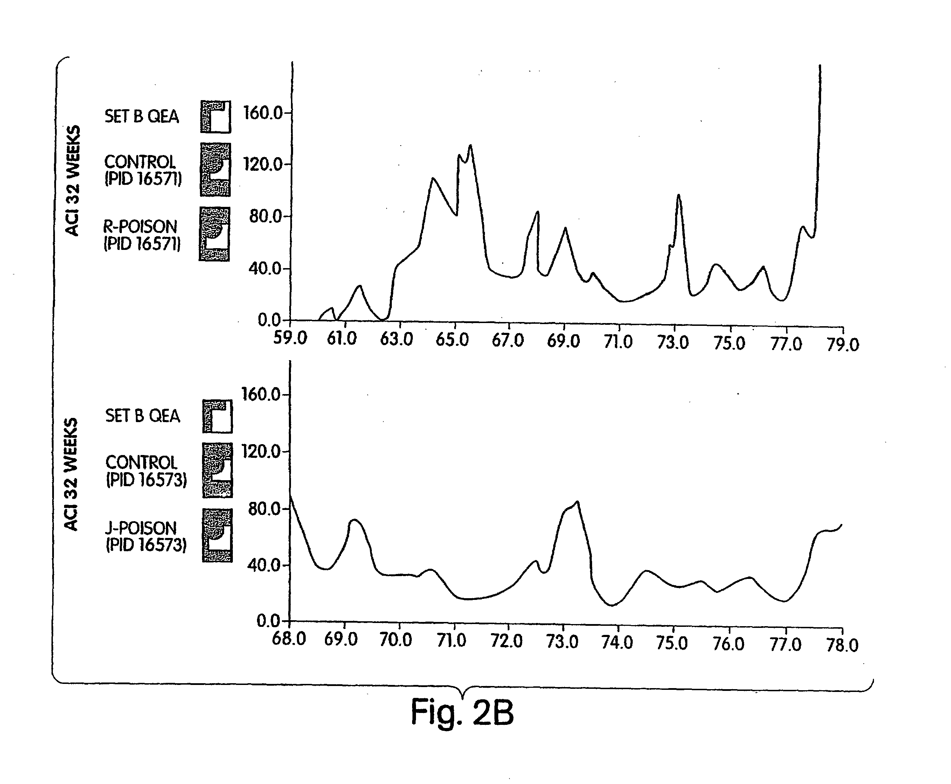

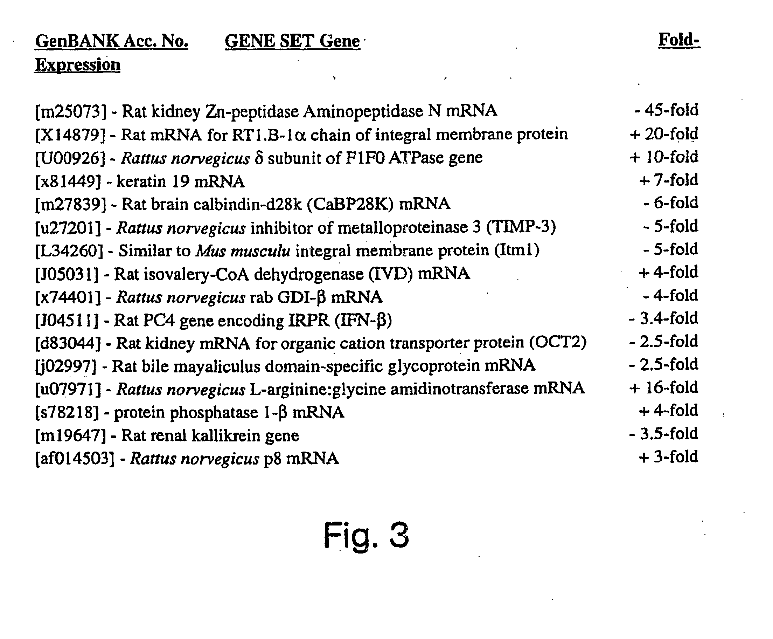

The confirmatory, Oligo-Poisoning™ results for the PC4 gene encoding IRPR (INF-β) is illustrated in Figure 2. .Analogous reactions were then performed for the remaining, differentially-expressed GENE SET genes/gene products.

### (II) Identification of GENE SET Genes by RNA Reverse Transcriptase Polymerase Chain Reaction (RT-PCR)

### (i) Tissue Dissection

Kidney tissue derived from the FHR, IRL and ACI (control) rodent strains may also be analyzed by the reverse transcriptase-polymerase chain reaction (RT-PCR) amplification methodology.

FHR, IRL and ACI rats were maintained on normal rat chow and water *ad libitum.* FHR and the control ACI rats were sacrificed at 1.5 and 7.5 months-of-age, whereas the IRL was sacrificed only at 1.5 months-of-age. While the 1.5 month rats appeared "normal" as a result of laboratory blood-work which was performed, the FHR by 7.5 months-of-age was demonstrated to have developed pronounced proteinuria. This finding is in agreement with previous experimental results which found that the median age of onset of proteinuria (and other renal-related disorders) in the FHR was approximately 4 months-of-age. Following the abnormal laboratory results indicating the onset of proteinuria, the animals were sacrificed and their kidneys removed and quick-frozen in liquid nitrogen immediately after dissection. The kidneys were stored at -70°C until utilized in the subsequent GeneCalling® protocols.

### (ii) Isolation of Total Cellular RNA

Total cellular RNA was extracted from 5 mg of heart, liver, fat, kidney, or brain tissue by initially grinding the tissue into a fine powder in liquid nitrogen. The powdered tissue was then transferred to a tube containing 500 µl Triazol Reagent® (Life Technologies; Gaithersburg, MD) and was dispersed using a Polytron homogenizer (Brinkman Instruments; Westbury, NY). See *e.g*., Chomszynski, *et al*. 1987. *Annal. Biochem.* 162 156-159; Chomszynski, *et al*., 1993. *Biotechniques* 15:532-533, 536-537. The total cellular RNA fraction was then extracted with 50 µl BCP (1-bromo-3-chloropropane; Molecular Research; Cincinnati, OH) to facilitate phase separation. The extraction mixture was centrifuged for 15 minutes at 4°C at 12,000 x G, and the aqueous phase was removed and transferred to a fresh tube. The RNA was then precipitated with 0.5 volume of isopropanol per original volume of Triazol Reagent® used, and the sample was re-centrifuged at room temperature for 10 minutes at 12,000 x G. The supernatant was then discarded, the pellet washed with 70% ethanol and re-centrifuged at room temperature for 5 minutes at 12,000 x G. Finally the 70% ethanol was removed and the centrifuge tube was inverted and let stand to dry in this position. The resulting RNA pellet was re-suspended in 100 µl water (*i.e*., 1 µl/mg of original tissue weight) and heated to 55°C until completely dissolved. The final concentration of total cellular RNA was quantitated by fluorometry with OliGreen® (Molecular Probes; Eugene, OR). In addition, the quality of the total cellular RNA was determined by both spectrophotometry and formaldehyde agarose gel electrophoresis. The total cellular RNA was stored at -20°C for subsequent utilization in the RT-PCR protocols.

### (iii) RNA Reverse Transcription (RT) Reactions

The initial reverse transcription reactions were performed as follows: 1 µg of total cellular RNA was mixed in 11 µl of RNase-free water (Ambion) with 1 µl (20:1 dilution; 100 pmoles/µl) oligo(dT)₂₅V primer (where V = A, C or G; Amitoff). Any RNA secondary structure was denatured by heating at 70°C for 10 minuets, followed by quick-chilling on ice. The denatured RNA was then collected by centrifugation for 15 seconds in a microfuge and to each tube was added: 4 µl 5X first-strand reaction buffer (BRL); 2 µl 0.1 mM DTT and 1 µl 10 mM dNTP mixture (Pharmacia). The reaction mixture was heated to 37°C for 2 minutes and 1 µl Superscript II reverse transcriptase (BRL) was added, followed by continued incubation at 37C for 1 hour.

### (iv) PCR-Mediated Amplification of the RT Products

Following reverse transcription, each of the samples were then subjected to PCR-mediated amplification. For a total of 10 samples, the following PCR reaction mixture was prepared: 50 µl 10X PCR buffer; 10µl dNTP mixture; 10 µl "sense" Primer (100 pmoles/µl; Pharmacia); 10 µl "anti-sense" Primer (100 pmoles/µl; Pharmacia); 2 µl KlenTaq (Life Technologies) and 418 µl RNase-free water. It should be noted that individuals skilled within the art may easily design both "sense" and "anti-sense" primers which would possess homology for the sequence(s) of interest. For the RT-PCR reactions, 49 µl of the PCR reaction mixture was added to 1 µl of the initial RT reaction and PCR amplification was performed for a total of 30 cycles in a thermal cycler under the following conditions: 96°C for 30 seconds; 57°C for 1 minute; 72°C for 2 minutes. The amplified products were then held at 4°C for subsequent analysis and agarose gel electrophoresis was performed to confirm the quality of the RT-PCR products.

Prior to further analysis, the RT-PCR products were subjected to a post-PCR clean up protocol as follows: MPG® streptavidin magnetic beads (CPG; Lincoln Park, NJ) were prepared (3 µl of beads for every 5 µl of RT-PCR reaction product) by pre-washing the beads in 10 µl binding buffer (5 M NaCl, 10 mM TRIS, pH 8.0, 1 mM EDTA) per 5 µl original volume of RT-PCR reaction product. 10 µl of washed beads were dispensed in a 96 well FALCON® TC plate for every RT-PCR sample processed. RT-PCR products were added to the beads, mixed well and incubated for 30 minutes at 50°C. The sample volume was made 100 µl with binding buffer, the plate placed on a 96 well magnetic particle concentrator, and the beads allowed to migrate for 5 minutes. The liquid was then removed, and 200 µl washing buffer (10 mM Tris, pH 7.4, 10 mM EDTA) added per well. The washing step was then repeated.

For analysis, the beads were resuspended in 5 µl loading buffer (80% deionized formamide, 20% 25 mM EDTA, pH 8.0, 50 mg/ml Blue Dextran) per 5 µl of beads, and the supernatant was then analyzed by electrophoresis on an ABI 377® (Applied Biosystems, Inc.) automated sequencer under denaturing conditions using the GeneSmay® computer software (ABI) for analysis. A GeneSmay 500® ROX ladder (diluted 1:10 in gel sample loading buffer) was utilized to a size control during the subsequent RT-PCR analysis.

### (B) Physiological and Biochemical Significance of the Experimental Results

A total of 20,000 gene fragments, generated from approximately 10,000 genes, were compared, in triplicate, between renal tissue samples derived from each of the three rat strains.
A comparison of previously-characterized, "common" differentially-expressed genes between the diseased animals (*i.e*., the FHR and IRL strains) and the control (*i.e*., ACI strain) animals across time points was then performed. A total of 36 gene fragments were identified using this technique.

Subsequently, further analysis was provided through the utilization of the GeneCalling® methodology. By use of the GeneCalling® methodology, a total of 16 genes (GENE SET) were found to be differentially-expressed among these rat strains. The differentially-expressed genes comprising the GENE SET are illustrated in Table 1 and include Zn-peptidase (Aminopeptidase N)

### The Zn-Peptidase (Aminopeptidase N)

As illustrated in Table 1 a 45-fold decrease in mRNA expression of the Zn-peptidase (Aminopeptidase N; GenBank Acc. No. Z25073) were found in the FHR and ACI animals, in comparison to the mRNA levels in the ACI control rodents.

The Zn-peptidase (Aminopeptidase N) is a cell surface peptidase composed of a single type of subunit with characteristics typical of ectoenzymes. Ectoenzymes are integral plasma membrane proteins with the majority of the molecule containing catalytic sites exposed to the external, non-cytoplasmic surface. See *e.g.,* Kenny & Turner, 1987. In: *Mammalian Ectoenzymes*, pp. 1-13 (Elsevier Scientific Publishing Co., Amsterdam). These enzymes, acting upon extracellular substrates, participate in the metabolism of secreted regulatory molecules and intestinal dietary substrates, as well as in the modulation of cell-cell interactions. See *e.g*., Luzio, *et al*., 1987. In: *Mammalian Ectoenzymes*, pp. 111-137 (Elsevier Scientific Publishing Co., Amsterdam).

The Zn-peptidase (Aminopeptidase N) is anchored in the cell membrane via a hydrophobic domain which is adjacent to a small cytoplasmic region at the amino-terminus of the protein (see *e.g.,* Feracci, *et al*., 1982. *Biochim. Biophys. Acta* 684:133-136) a short "stalk-like" projection connects the transmembranal domain to the hydrophilic, extracellular region which comprises the majority of the molecule (see *e.g*., Hussain, *et al*., 1981. *Biochem. J*. 199:179-186). The catalytic activity of Zn-peptidase (Aminopeptidase N), which preferentially removes neutral amino-terminus amino acid residues from oligopeptides, is present in the extracellular region. See *e.g.,* Louvard, *et al*., 1975. *Biochim. Biophys*. *Acta* 389:389-400. Zn-peptidase (Aminopeptidase N) has been demonstrated to be widely distributed in numerous tissue (including the central nervous system) and is particularly abundant in the kidney and intestinal microvilli. In a comparison of numerous tissues in the rat, Zn-peptidase (Aminopeptidase N) transcripts were found to be approximately 5-fold higher in the kidney than in the next most abundant tissue, the lung. See *e.g.,* Watt & Yip, 1989. *J. Biol. Chem.* 264:5480-5487. These findings have led to the proposal that Aminopeptidase N functions to cleave dietary substrates prior to absorption in the intestine and to regulate the action of hormones and neurotransmitters by inactivating such peptides at the cell surface (see *e.g*., Turner, *et al*., 1987. In: *Mammalian Ectoenzymes*, pp. 211-248 (Elsevier Scientific Publishing Co., Amsterdam).

Human Zn-peptidase (Aminopeptidase N) has been shown to be encoded by a single gene (designated KZP), localized on chromosome 15. See *e.g.,* Watt & Yip, 1989. *J. Biol. Chem.* 264:5480-5487. In several mammalian species, including humans, two mRNAs of approximately 3.4 and 3.9 Kb, whose relative concentrations varied among different tissues, have been demonstrated. These multiple transcripts do not appear to be due to the presence of a pseudogene. The difference in mRNA size may be due either to: (*i*) varying lengths of 3'- or 5'-intronic region(s) or (*ii*) alternative splicing of a primary transcript from the same gene.

The 45-fold decrease in Zn-peptidase (Aminopeptidase N) mRNA expression in the FHR and IRL animals is interesting due to the normally high levels of this transcript in the kidney. As disclosed by the present invention, the FHR and IRL rat strains possess a dramatic decrease in the expression of the Zn-peptidase (Aminopeptidase N) transcripts. Accordingly, exogenous administration of this enzyme may function to ameliorate some of the deleterious physiological effects of renal disease and/or associated disorders. Additionally, quantitation of the level of Zn-peptidase (Aminopeptidase N) mRNAs may be useful in prognostic tests for a predisposition to kidney disease or in the diagnosis of early/sub-clinical renal disease or associated disorders.

WO-A-94/16724 describes the prevention and treatment of cytomegalovirus (CMV) infection using human Aminopeptidase N.

## Claims

1. Use of Zn-peptidase(Aminopeptidase N) in the manufacture of a medicament for treating or preventing renal disease.

2. Use according to claim 1, wherein the Zn-peptidase (Aminopeptidase N) is human.

3. Use of one or more of the following:
(a) antibodies specific for the Zn-peptidase (Aminopeptidase N) of claim 1;
(b) nucleic acids which encode the Zn-peptidase (Aminopeptidase N) of claim 1;
(c) anti-sense nucleic acid derivatives of the nucleic acids encoding the Zn-peptidase (Aminopeptidase N) of claim 1;
(d) antibodies specific for one or more of the nucleic acids which encode the Zn-peptidase (Aminopeptidase N) of claim 1; and
(e) antibodies specific for the anti-sense nucleic acid derivatives of the nucleic acids encoding the Zn-peptidase (Aminopeptidase N) of claim 1,
in the manufacture of a medicament for treating or preventing renal disease.

4. Use of one or more of the following:
(a) antibodies specific for the Zn-peptidase (Aminopeptidase N) of claim 1;
(b) nucleic acids which encode the Zn-peptidase (Aminopeptidase N) of claim 1;
(c) anti-sense nucleic acid derivatives of the nucleic acids encoding the Zn-peptidase (Aminopeptidase N) of claim 1;
(d) antibodies specific for one or more of the nucleic acids which encode the Zn-peptidase (Aminopeptidase N) of claim 1; and
(e) antibodies specific for the anti-sense nucleic acid derivatives of the nucleic acids encoding the Zn-peptidase (Aminopeptidase N) of claim 1,
in the manufacture of a medicament for treating or preventing platelet storage-pool disease.

5. Use of one or more of the following:
(a) antibodies specific for the Zn-peptidase (Aminopeptidase N) of claim 1;
(b) nucleic acids which encode the Zn-peptidase (Aminopeptidase N) of claim 1;
(c) anti-sense nucleic acid derivatives of the nucleic acids encoding Zn-peptidase (Aminopeptidase N) of claim 1;
(d) antibodies specific for one or more of the nucleic acids which encode the Zn-peptidase (Aminopeptidase N) of claim 1; and
(e) antibodies specific for the anti-sense nucleic acid derivatives of the nucleic acids encoding the Zn-peptidase (Aminopeptidase N) of claim 1,
in the manufacture of a medicament for treating or preventing hypertension.

6. A pharmaceutical composition for the treatment of prevention of renal disease comprising a therapeutically or prophylactically effective amount of one or more of the following:
(a) an isolated, Zn-peptidase (Aminopeptidase N) protein as recited in claim 1;
(b) antibodies specific for the Zn-peptidase (Aminopeptidase N) of claim 1;
(c) nucleic acids encoding the Zn-peptidase (Aminopeptidase N) of claim 1;
(d) anti-sense nucleic acid derivatives of the nucleic acids encoding the Zn-peptidase (Aminopeptidase N) of claim 1;
(e) antibodies specific for one or more of said nucleic acids which encode the Zn-peptidase (Aminopeptidase N) of claim 1; and
(f) antibodies specific for one or more of said anti-sense nucleic acid derivatives of the Zn-peptidase (Aminopeptidase N) of claim 1,
and a pharmaceutically acceptable carrier.

7. A kit, comprising in one or more containers, a therapeutically or prophylactically effective amount of the pharmaceutical composition of claim 6.

8. The pharmaceutical composition of claim 6, wherein said nucleic acid is a nucleic acid vector.

9. The pharmaceutical composition of claim 6, wherein said anti-sense nucleic acid derivative is a nucleic acid vector.

10. A method of screening for a modulator of renal disease, platelet storage-pool disease, hypertension or related diseases or disorders of Zn-peptidase (Aminopeptidase N) said method comprising:
(a) contacting a cell with a test compound in vitro;
(b) measuring the levels of Zn-peptidase (Aminopeptidase N) in said cell;
(c) measuring the level of Zn-peptidase (Aminopeptidase N) in a control cell not contacted with said test compound; and
(d) comparing the levels of Zn-peptidase (Aminopeptidase N) in the cells of step (b) and (c), wherein an alteration in the level of proteins in the cells indicates that the test compound is a modulator of renal disease, platelet storage-pool disease, hypertension and/or related diseases or disorders.

11. A method of screening for a modulator of renal disease, platelet storage-pool disease, hypertension or related diseases or disorders, wherein:
(a) a test compound has been administered to a non-human test animal which is predisposed to developing or has already developed renal disease, platelet storage-pool disease, hypertension or related diseases or disorders;
(b) the test compound has been administered to a matched control non-human animal which is predisposed to developing or has already developed renal disease, platelet storage-pool disease, hypertension or related diseases or disorders;
said method comprising:
(c) measuring the level of the Zn-peptidase (Aminopeptidase N) protein or nucleic acid or a nucleic acid encoding the nucleic acid in the animals of step (a) and (b); and
(d) comparing the levels of the protein or nucleic acid in the test and matched control animals,
wherein a change the relative levels indicates that the test compound is a modulator of renal disease, platelet storage-pool disease, hypertension and/or related diseases or disorders.

12. The method of claim 11, wherein said non-human test animal is a recombinant non-human test animal which expresses a Zn-peptidase (Aminopeptidase N) transgene or expresses Zn-peptidase (Aminopeptidase N) under the control of a promoter which is not the native Zn-peptidase (Aminopeptidase N) gene promoter at an increased level relative to a wild-type test animal.

13. A recombinant, non-human animal comprising a non-native Zn-peptidase (Aminopeptidase N) gene sequence.

14. Use according to any one of claims 1-5 wherein said medicament further comprises a drug selected from the group consisting of:
(a) a sympatholytic;
(b) an angiotensin inhibiting drug;
(c) a calcium channel blocking drug;
(d) a diurectic; and
(e) a vasodilator.

15. A method useful for diagnosing the susceptibility or presence of renal disease, platelet storage-pool disease, hypertension or related diseases or disorders comprising:
(a) having provided a tissue sample from a subject;
(b) measuring the level of Zn-peptidase (Aminopeptidase N) in said subject or measuring the level of a nucleic acid encoding Zn-peptidase (Aminopeptidase N) in said subject; and
(c) comparing the amount of protein or nucleic acid in said test sample with the level of protein or nucleic acid in a control sample, wherein the control sample is taken from an individual not suffering or susceptible to platelet storage-pool disease, hypertension or related diseases or disorders,
wherein an alteration in the level of the protein or nucleic acid in the subject relative to the control indicates the subject is susceptible to or suffers from renal disease, platelet storage-pool disease, hypertension or related diseases or disorders.

16. The method of claim 15, wherein said Zn-peptidase (Aminopeptidase N) is measured using an antibody for said protein.

## Patentansprüche

1. Verwendung von Zn-Peptidase (Aminopeptidase N) für die Herstellung eines Medikamentes zur Behandlung oder Vorbeugung von Nierenerkrankung.

2. Verwendung nach Anspruch 1, worin die Zn-Peptidase (Aminopeptidase N) menschlich ist.

3. Verwendung von einem oder mehr der Folgenden:
a) Antikörpern, die spezifisch für die Zn-Peptidase (Aminopeptidase N) von Anspruch 1 sind;
b) Nukleinsäuren, welche die Zn-Peptidase (Aminopeptidase N) von Anspruch 1 kodieren;
c) Antisense-Nukleinsäurederivaten der Nukleinsäuren, die die Zn-Peptidase (Aminopeptidase N) von Anspruch 1 kodieren;
d) Antikörpern, die spezifisch für eine oder mehrere der Nukleinsäuren, die die Zn-Peptidase (Aminopeptidase N) von Anspruch 1 kodieren, sind; und
e) Antikörpern, die spezifisch für die Antisense-Nukleinsäurederivate der Nukleinsäuren, die die Zn-Peptidase (Aminopeptidase N) nach Anspruch 1 kodieren, sind,
für die Herstellung eines Medikamentes zur Behandlung oder Vorbeugung von Nierenerkrankung.

4. Verwendung von einem oder mehr der Folgenden:
a) Antikörpern, die spezifisch für die Zn-Peptidase (Aminopeptidase N) von Anspruch 1 sind;
b) Nukleinsäuren, welche die Zn-Peptidase (Aminopeptidase N) von Anspruch 1 kodieren;
c) Antisense-Nukleinsäurederivaten der Nukleinsäuren, die die Zn-Peptidase (Aminopeptidase N) von Anspruch 1 kodieren;
d) Antikörpern, die spezifisch für eine oder mehrere der Nukleinsäuren, die die Zn-Peptidase (Aminopeptidase N) von Anspruch 1 kodieren, sind; und
e) Antikörpern, die spezifisch für die Antisense-Nukleinsäurederivate der Nukleinsäuren, die die Zn-Peptidase (Aminopeptidase N) nach Anspruch 1 kodieren, sind,
für die Herstellung eines Medikamentes zur Behandlung oder Vorbeugung von Thrombozytenspeicherkrankheit (platelet storage-pool disease).

5. Verwendung von einem oder mehr der Folgenden:
a) Antikörpern, die spezifisch für die Zn-Peptidase (Aminopeptidase N) von Anspruch 1 sind;
b) Nukleinsäuren, welche die Zn-Peptidase (Aminopeptidase N) von Anspruch 1 kodieren;
c) Antisense-Nukleinsäurederivaten der Nukleinsäuren, die die Zn-Peptidase (Aminopeptidase N) von Anspruch 1 kodieren;
d) Antikörpern, die spezifisch für eine oder mehrere der Nukleinsäuren, die die Zn-Peptidase (Aminopeptidase N) von Anspruch 1 kodieren, sind; und
e) Antikörpern, die spezifisch für die Antisense-Nukleinsäurederivate der Nukleinsäuren, die die Zn-Peptidase (Aminopeptidase N) nach Anspruch 1 kodieren, sind,
für die Herstellung eines Medikamentes zur Behandlung oder Vorbeugung von Hypertonie bzw. Bluthochdruck.

6. Pharmazeutische Zubereitung für die Behandlung oder Vorbeugung von Nierenerkrankung, die eine therapeutisch oder prophylaktisch wirksame Menge von einem oder mehreren der Folgenden umfasst:
a) ein isoliertes Zn-Peptidase- (Aminopeptidase N)-Protein, wie in Anspruch 1 angeführt;
b) Antikörper, die spezifisch für die Zn-Peptidase (Aminopeptidase N) von Anspruch 1 sind;
c) Nukleinsäuren, die die Zn-Peptidase (Aminopeptidase N) von Anspruch 1 kodieren;
d) Antisense-Nukleinsäurederivate der Nukleinsäuren, die die Zn-Peptidase (Aminopeptidase N) von Anspruch 1 kodieren;
e) Antikörper, die spezifisch für eine oder mehrere dieser Nukleinsäuren, die die Zn-Peptidase (Aminopeptidase N) von Anspruch 1 kodieren, sind;
f) Antikörper, die spezifisch für eine oder mehrere dieser Antisense-Nukleinsäurederivate der Zn-Peptidase (Aminopeptidase N) von Anspruch 1 sind,
und einen pharmazeutisch annehmbaren Träger.

7. Ein Kit, der in einem oder mehreren Behältern eine therapeutisch oder prophylaktisch wirksame Menge der pharmazeutischen Zubereitung nach Anspruch 6 umfasst.

8. Pharmazeutische Zubereitung nach Anspruch 6, worin diese Nukleinsäure ein Nukleinsäurevektor ist.

9. Pharmazeutische Zusammensetzung nach Anspruch 6, worin das Antisense-Nukleinsäurederivat ein Nukleinsäurevektor ist.

10. Screeningverfahren für einen Modulator von Nierenerkrankung, Thrombozytenspeichererkrankung (platelet storage-pool disease), Hypertonie oder verwandten Krankheiten oder Störungen der Zn-Peptidase (Aminopeptidase N), wobei dieses Verfahren umfasst:
a) Kontaktieren einer Zelle mit einer Testsubstanz in vitro;
b) Messen des Spiegels der Zn-Peptidase (Aminopeptidase N) in dieser Zelle;
c) Messen des Spiegels der Zn-Peptidase (Aminopeptidase N) in einer Kontrollzelle, welche nicht mit dieser Testsubstanz kontaktiert wurde; und
d) Vergleichen der Spiegel der Zn-Peptidase (Aminopeptidase N) in den Zellen von Schritt (b) und (c), wobei eine Änderung des Spiegels der Proteine in den Zellen anzeigt, dass die Testsubstanz ein Modulator von Nierenerkrankung, Thrombozytenspeichererkrankung, Hypertonie und/oder verwandten Krankheiten oder Störungen ist.

11. Screeningverfahren für einen Modulator von Nierenerkrankung, Thrombozytenspeichererkrankung (platelet storage-pool disease), Hypertonie oder verwandten Krankheiten oder Störungen, worin:
a) eine Testsubstanz einem nicht menschlichen Versuchstier verabreicht worden ist, welches prädisponiert ist, Nierenerkrankung, Thrombozytenspeichererkrankung, Hypertonie oder verwandte Krankheiten oder Störungen zu entwickeln, oder bereits entwickelt hat;
b) die Testsubstanz einem passenden, nicht menschlichen Kontrolltier verabreicht worden ist, welches prädisponiert ist, Nierenerkrankung, Thrombozytenspeichererkrankung, Hypertonie oder verwandte Krankheiten oder Störungen zu entwickeln, oder bereits entwickelt hat; und dieses Verfahren umfasst;
c) Messen des Spiegels des Zn-Peptidase- (Aminopeptidase N)-Proteins oder der -Nukleinsäure oder einer Nukleinsäure, die die Nukleinsäure in den Tieren von Schritt (a) und (b) kodiert; und
d) Vergleichen der Spiegel des Proteins oder der Nukleinsäuren in den Versuchstieren und den passenden Kontrolltieren,
wobei eine Änderung der relativen Spiegel anzeigt, dass die Testsubstanz ein Modulator von Nierenerkrankung, Thrombozytenspeichererkrankung, Hypertonie und/oder verwandten Krankheiten oder Störungen ist.

12. Verfahren nach Anspruch 11, wobei dieses nicht menschliche Versuchstier ein rekombinantes, nicht menschliches Versuchstier ist, welches ein Zn-Peptidase- (Aminopeptidase N)-Transgen exprimiert oder welches Zn-Peptidase (Aminopeptidase N) exprimiert unter der Kontrolle eines Promoters, der nicht der natürliche Zn-Peptidase- (Aminopeptidase N)-Genpromoter ist, mit einem erhöhten Spiegel im Vergleich zu einem Wildtyp-Versuchstier.

13. Rekombinantes, nicht menschliches Tier, das eine nicht natürliche Zn-Peptidase-(Aminopeptidase N)-Gensequenz enthält.

14. Verwendung nach einem der Ansprüche 1-5, worin dieses Medikament weiterhin einen Wirkstoff umfasst, ausgewählt aus der Gruppe bestehend aus:
a) einem Sympatholytikum;
b) einem Angiotensin inhibierenden Wirkstoff;
c) einem Calciumkanal hemmenden Wirkstoff;
d) einem Diuretikum; und
e) einem Vasodilatator.

15. Verfahren, nützlich in der Diagnose der Empfänglichkeit für oder des Vorhandenseins von Nierenerkrankung, Thrombozytenspeichererkrankung (platelet-storage pool disease), Hypertonie oder verwandten Krankheiten oder Störungen, welches umfasst:
a) Bereitgestellt haben einer Gewebeprobe von einem Subjekt;
b) Messen des Spiegels der Zn-Peptidase (Aminopeptidase N) in diesem Subjekt oder Messen des Spiegels einer Nukleinsäure, die Zn-Peptidase (Aminopeptidase N) kodiert, in diesem Subjekt; und
c) Vergleichen der Menge an Protein oder Nukleinsäure in dieser Testprobe mit dem Spiegel an Protein oder Nukleinsäure in einer Kontrollprobe, wobei die Kontrollprobe von einem Individuum entnommen wird, das nicht an Thrombozytenspeichererkrankung, Hypertonie oder verwandten Krankheiten oder Störungen leidet oder dafür empfänglich ist,
wobei eine Änderung in dem Spiegel des Proteins oder der Nukleinsäure in dem Subjekt im Vergleich zu der Kontrolle anzeigt, dass das Subjekt empfänglich ist für oder leidet an Nierenerkrankung, Thrombozytenspeichererkrankung, Hypertonie oder verwandten Krankheiten oder Störungen.

16. Verfahren nach Anspruch 15, wobei diese Zn-Peptidase (Aminopeptidase N) unter Verwendung eines Antikörpers für dieses Protein gemessen wird.

## Revendications

1. Utilisation de la Zn-peptidase (Aminopeptidase N) pour la fabrication d'un médicament destiné à la thérapie ou à la prévention de maladies rénales.

2. Utilisation selon la revendication 1, dans laquelle la Zn-peptidase (Aminopeptidase N) est humaine.

3. Utilisation de l'un ou de plusieurs des éléments suivants :
(a) anticorps spécifiques pour la Zn-peptidase (Aminopeptidase N) de la revendication 1 ;
(b) acides nucléiques qui codent la Zn-peptidase (Aminopeptidase N) de la revendication 1 ;
(c) dérivés d'acide nucléique antisens des acides nucléiques qui codent la Zn-peptidase (Aminopeptidase N) de la revendication 1 ;
(d) anticorps spécifiques pour l'un ou plusieurs des acides nucléiques qui codent la Zn-peptidase (Aminopeptidase N) de la revendication 1 ; et
(e) anticorps spécifiques pour les dérivés d'acide nucléique antisens des acides nucléiques qui codent la Zn-peptidase (Aminopeptidase N) de la revendication 1, pour la fabrication d'un médicament destiné à la thérapie ou à la prévention de maladies rénales

4. Utilisation de l'un ou de plusieurs des éléments suivants :
(a) anticorps spécifiques pour la Zn-peptidase (Aminopeptidase N) de la revendication 1 ;
(b) acides nucléiques qui codent la Zn-peptidase (Aminopeptidase N) de la revendication 1 ;
(c) dérivés d'acide nucléique antisens des acides nucléiques qui codent la Zn-peptidase (Aminopeptidase N) de la revendication 1 ;
(d) anticorps spécifiques pour l'un ou plusieurs des acides nucléiques qui codent la Zn-peptidase (Aminopeptidase N) de la revendication 1 ; et
(e) anticorps spécifiques pour les dérivés d'acide nucléique antisens des acides nucléiques qui codent la Zn-peptidase (Aminopeptidase N) de la revendication 1, pour la fabrication d'un médicament destiné à la thérapie ou à la prévention de la maladie du stockage plaquettaire.

5. Utilisation de l'un ou de plusieurs des éléments suivants :
(a) anticorps spécifiques pour la Zn-peptidase (Aminopeptidase N) de la revendication 1 ;
(b) acides nucléiques qui codent la Zn-peptidase (Aminopeptidase N) de la revendication 1 ;
(c) dérivés d'acide nucléique antisens des acides nucléiques qui codent la Zn-peptidase (Aminopeptidase N) de la revendication 1 ;
(d) anticorps spécifiques pour l'un ou plusieurs des acides nucléiques qui codent la Zn-peptidase (Aminopeptidase N) de la revendication 1 ; et
(e) anticorps spécifiques pour les dérivés d'acide nucléique antisens des acides nucléiques qui codent la Zn-peptidase (Aminopeptidase N) de la revendication 1, pour la fabrication d'un médicament destiné à la thérapie ou à la prévention de l'hypertension.

6. Composition pharmaceutique pour la thérapie ou la prévention de maladies rénales comprenant une quantité thérapeutiquement ou prophylactiquement efficace pour la thérapie ou la prophylaxie de l'un ou de plusieurs des éléments suivants :
(a) une protéine isolée de la Zn-peptidase (Aminopeptidase N) selon la revendication 1 ;
(b) anticorps spécifiques pour la Zn-peptidase (Aminopeptidase N) de la revendication 1 ;
(c) acides nucléiques qui codent la Zn-peptidase (Aminopeptidase N) de la revendication 1 ;
(d) dérivés d'acide nucléique antisens des acides nucléiques qui codent la Zn-peptidase (Aminopeptidase N) de la revendication 1 ;
(e) anticorps spécifiques pour l'un ou plusieurs desdits acides nucléiques qui codent la Zn-peptidase (Aminopeptidase N) de la revendication 1 ; et
(f) anticorps spécifiques pour l'un ou plusieurs desdits dérivés d'acide nucléique antisens des acides nucléiques qui codent la Zn-peptidase (Aminopeptidase N) de la revendication 1,
et un véhicule pharmaceutiquement acceptable.

7. Kit comprenant, dans un ou plusieurs récipients, une quantité thérapeutiquement ou prophylactiquement efficace de la composition pharmaceutique selon la revendication 6.

8. Composition pharmaceutique selon la revendication 6, dans laquelle ledit acide nucléique est un acide nucléique vecteur.

9. Composition pharmaceutique selon la revendication 6, dans laquelle ledit dérivé d'acide nucléique antisens est un acide nucléique vecteur.

10. Procédé de criblage pour un modulateur de maladies rénales, de la maladie du stockage plaquettaire, de l'hypertension ou des maladies ou troubles associés à la Zn-peptidase (Aminopeptidase N), ledit procédé comprenant :
(a) la mise en contact d'une cellule avec un composé test in vitro ;
(b) la mesure des niveaux de Zn-peptidase (Aminopeptidase N) dans ladite cellule ;
(c) la mesure du niveau de Zn-peptidase (Aminopeptidase N) dans une cellule témoin qui n'a pas été mise en contact avec ledit composé test ; et
(d) la comparaison des niveaux de Zn-peptidase (Aminopeptidase N) dans les cellules des étapes (b) et (c), dans lesquelles une altération du niveau de protéines dans les cellules indique que le composé test est un modulateur de maladies rénales, de la maladie du stockage plaquettaire, de l'hypertension et/ou des maladies ou troubles associés.

11. Procédé de criblage pour un modulateur de maladies rénales, de la maladie du stockage plaquettaire, de l'hypertension et/ou des maladies ou troubles associés, dans lequel :
(a) un composé test a été administré à un animal test non humain qui est prédisposé à développer ou qui a déjà développé une maladie rénale, la maladie du stockage plaquettaire, de l'hypertension et/ou des maladies ou troubles associés ;
(b) le composé test a été administré à un animal non humain témoin correspondant qui est prédisposé à développer ou qui a déjà développé une maladie rénale, la maladie du stockage plaquettaire, de l'hypertension et/ou des maladies ou troubles associés ; ledit procédé comprenant :
(c) la mesure du niveau de protéine ou d'acide nucléique de Zn-peptidase (Aminopeptidase N) ou d'un acide nucléique codant l'acide nucléique chez les animaux des étapes (a) et (b) ; et
(d) la comparaison des niveaux de protéine ou d'acide nucléique chez les animaux test et témoin correspondant,
dans lequel une modification des niveaux relatifs indique que le composé test est un modulateur de maladies rénales, de la maladie du stockage plaquettaire, de l'hypertension et/ou des maladies ou troubles associés.

12. Procédé selon la revendication 11, dans lequel ledit animal test non humain est un animal test non humain recombinant qui exprime un transgène de la Zn-peptidase (Aminopeptidase N) ou qui exprime la Zn-peptidase (Aminopeptidase N) sous le contrôle d'un promoteur qui n'est pas le promoteur du gène de la Zn-peptidase (Aminopeptidase N) natif, à un niveau accru par rapport à un animal test de type sauvage.

13. Animal test recombinant non humain comprenant une séquence génique de Zn-peptidase (Aminopeptidase N) non native.

14. Utilisation selon l'une quelconque des revendications 1 à 5 dans laquelle ledit médicament comprend en outre un médicament choisi dans le groupe consistant en :
(a) un sympatholytique ;
(b) un inhibiteur de l'angiotensine ;
(c) un médicament bloquant le canal calcium ;
(d) un diurétique ; et
(e) un vasodilatateur.

15. Procédé utile pour le diagnostic de la susceptibilité ou la présence de maladies rénales, à la maladie du stockage plaquettaire, à l'hypertension et/ou aux maladies ou troubles associés, comprenant :
(a) le prélèvement d'un échantillon de tissu d'un sujet ;
(b) la mesure du niveau de Zn-peptidase (Aminopeptidase N) chez ledit sujet ou la mesure du niveau d'un acide nucléique codant la Zn-peptidase (Aminopeptidase N) chez ledit sujet ; et
(c) la comparaison de la quantité de protéine ou d'acide nucléique dans ledit échantillon test et de la quantité de protéine ou d'acide nucléique dans un échantillon témoin, dans lequel l'échantillon témoin est prélevé chez un individu ne souffrant pas de ou n'étant pas susceptible à la maladie du stockage plaquettaire, l'hypertension et/ou des maladies ou troubles associés,
dans lequel une altération du niveau de protéine ou d'acide nucléique chez le sujet par rapport au témoin indique que le sujet est sensible à, ou souffre d'une maladie rénale, de la maladie du stockage plaquettaire, d'hypertension et/ou de maladies ou troubles associés.

16. Procédé selon la revendication 15, dans lequel ladite Zn-peptidase (Aminopeptidase N) est mesurée en utilisant un anticorps pour ladite protéine.
